(19) Europäisches Patentamt
European Patent Office
Office européen des brevets

(11) **EP 4 140 427 B1**

(12) **EUROPÄISCHE PATENTSCHRIFT**

(45) Veröffentlichungstag und Bekanntmachung des Hinweises auf die Patenterteilung:
**24.07.2024 Patentblatt 2024/30**

(21) Anmeldenummer: **22192317.0**

(22) Anmeldetag: **26.08.2022**

(51) Internationale Patentklassifikation (IPC):
***A61B 18/12*** *(2006.01)* *A61B 18/00* *(2006.01)*

(52) Gemeinsame Patentklassifikation (CPC):
**A61B 18/1206;** A61B 2018/00607; A61B 2018/1273

(54) **ELEKTROCHIRURGIE-GENERATOR MIT DYNAMIKVERBESSERTEM INVERTER**

ELECTROSURGICAL GENERATOR WITH INVERTER WITH IMPROVED DYNAMICS

GÉNÉRATEUR D'ÉLECTROCHIRURGIE POURVU D'INVERSEUR À DYNAMIQUE AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.08.2021 US 202163237395 P**

(43) Veröffentlichungstag der Anmeldung:
**01.03.2023 Patentblatt 2023/09**

(73) Patentinhaber: **Olympus Winter & Ibe GmbH 22045 Hamburg (DE)**

(72) Erfinder:
• **Dijkstra, Jelle 10781 Berlin (DE)**
• **Fähsing, Thomas 12305 Berlin (DE)**
• **Ramin, Daniel 14558 Nuthetal (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
EP-A1- 3 216 409 EP-A2- 2 779 410
WO-A1-2017/152181 DE-B3- 102013 005 277

**Beschreibung**

Die Erfindung betrifft einen Elektrochirurgie-Generator, der dazu ausgebildet ist eine hochfrequente Wechselspannung an ein elektrochirurgisches Instrument abzugeben. Er umfasst eine Gleichspannungsversorgung und einen Inverter für Hochspannung, der von der Gleichspannungsversorgung gespeist ist und eine hochfrequente Wechselspannung erzeugt, die an einem Ausgang zum Anschluss des elektrochirurgischen Instruments angelegt ist.

In der Elektrochirurgie wird hochfrequenter Wechselstrom verwendet insbesondere zum Schneiden bzw. Durchtrennen von Gewebe sowie zur Entfernung von Körpergewebe im Sinne einer thermischen Resektion (sogenanntes elektrisches Skalpell). Das Funktionsprinzip beruht auf Erwärmung des zu schneidenden Gewebes. Ein Vorteil liegt darin, dass gleichzeitig mit dem Schnitt auch eine Blutungsstillung durch Verschluss der betroffenen Gefäße erfolgen kann (Koagulation). Hierzu werden beträchtliche Leistungen benötigt, und zwar bei Frequenzen von 200 kHz oder höher bis zu 4000 kHz, typischerweise um die 400 kHz. Bei solchen Frequenzen verhält sich das Körpergewebe wie ein ohmscher Widerstand. Der spezifische Widerstand hängt jedoch stark von der Gewebeart ab, so unterscheiden sich die spezifischen Widerstände von Muskeln, Fett oder Knochen stark voneinander, und zwar bis zum Faktor 1000. Dies führt dazu, dass sich im Betrieb die Lastimpedanz des elektrischen Skalpells abhängig von dem zu schneidenden Gewebe schnell und stark ändern kann, bis hin zu einem Nahezu-Kurzschluss. Das stellt besondere und einzigartige Anforderungen an den Elektrochirurgie-Generator und dessen Hochspannungsbereitstellung. Insbesondere ist eine schnelle Spannungsregelung erforderlich, geeignet für hohe Spannungen im Bereich von einigen Kilovolt und hohe Frequenz in einem weiten Bereich von typischerweise zwischen 200 kHz und bis zu 4 MHz.

Je nach Gewebe und der sich daraus ergebenden Impedanz variieren die Ströme zwischen einigen Milliampere und mehreren Ampere, und zwar hochdynamisch innerhalb kürzester Zeit. Die Wellenform der abgegebenen Wechselspannung kann kontinuierlich sinusförmig sein oder kann moduliert sein mit einem Scheitelfaktor von bis zu 10 bei Modulationsfrequenzen von bis zu etwa 20 kHz.

Herkömmliche Elektrochirurgie-Generatoren sind häufig nach dem Prinzip eines Eintakt-Hochspannungswandlers aufgebaut. Sie weisen einen Wechselrichter zur Versorgung des elektrochirurgischen Instruments auf, dem gleichgerichteter Strom aus dem Netz mit unterschiedlicher Spannung zugeführt wird. Dazu ist erforderlich, dass die DC-Versorgung bezüglich der gelieferten Gleichspannung verstellbar ist. Der Wechselrichter ist typischerweise ausgeführt als freischwingender Eintaktgenerator mit einem LC-Resonanzkreis (beispielhaft: EP 2514380 B1).

Diese Bauart ist in der Praxis bewährt, weist aber auch Nachteile auf. Zum einen ist aufgrund hoher Verluste die Effizienz gering. Zudem treten große Blindströme im Resonanzkreis auf, was größer dimensionierte Komponenten erforderlich macht und zusätzlich den Wirkungsgrad bei niedriger Leistung verschlechtert. Ferner ist die Ausgangsfrequenz lastabhängig, ebenso wie der Scheitelfaktor, was ungünstig ist für stark modulierte Moden. Die Regelung der Ausgangsspannung ist verhältnismäßig langsam, so dass die Anpassung an veränderte Lastimpedanzen nur schlecht ist. Ein weiteres Beispiel eines Elektrochirurgie-Generators wird im EP 3216409 A1 offenbart.

Zur besseren Erfüllung dieser einzigartigen Anforderungen ist von der Anmelderin ein bisher unveröffentlichtes neuartiges Konzept für Elektrochirurgie-Generatoren unter Verwendung eines Multilevel-Inverters, entwickelt worden. Dieser ermöglicht eine verbesserte gesteuerte Erzeugung und Abgabe der Wechselspannung für das elektrochirurgische Instrument. Eine aufwendige spannungsveränderliche DC-Versorgung ist hierbei nicht mehr erforderlich, sondern die Höhe (sowie Frequenz und Wellenform) der abgegebenen Wechselspannung wird direkt über den Multilevel-Inverter gesteuert. Allerdings ist der Dynamikumfang begrenzt, und zwar insbesondere bei kleinerer abgegebener Wechselspannung-Amplitude. Der erreichbare Modulationsindex verringert sich mit abnehmender Ausgangsspannung. Dies begrenzt nicht nur die Fähigkeit zur Abgabe von modulierten Signalformen (sogenannten Moden), sondern verschlechtert ggf. auch die Wellenform der erzeugten Wechselspannung.

Dem könnte dadurch begegnet werden, dass die Höhe der Gleichspannungsversorgung des Inverters veränderlich gemacht wird. Allerdings gibt man damit den Vorteil einer spannungskonstanten Versorgung und der hiermit einhergehenden Vereinfachung der Gleichspannungsversorgung aus der Hand.

Der Erfindung liegt die Aufgabe zugrunde, einen Elektrochirurgie-Generator bereitzustellen, der einen höheren Dynamikumfang bereitstellt, ohne auf eine in der Spannungshöhe veränderliche Gleichspannungsversorgung angewiesen zu sein.

Die erfindungsgemäße Lösung liegt in den Merkmalen des unabhängigen Anspruchs. Vorteilhafte Weiterbildungen sind Gegenstand der abhängigen Ansprüche.

Bei einem Elektrochirurgie-Generator, der ausgebildet ist eine hochfrequente Wechselspannung an ein elektrochirurgisches Instrument abzugeben, umfassend eine Gleichspannungsversorgung und einen Inverter für Hochspannung, der von der Gleichspannungsversorgung gespeist ist und eine hochfrequente Wechselspannung mit variabler Spannung und Frequenz erzeugt, die an einem Ausgang zum Anschluss des elektrochirurgischen Instruments angelegt ist, ist erfindungsgemäß vorgesehen, dass der Inverter ausgeführt ist als ein von einem Referenzsignal für die abzugebende Spannung gesteuerter Multilevel-Inverter mit mindestens zwei Gruppen von in Reihe geschalteten Wechselrichter-Zellen, wobei jede Gruppe mit einer anderen Gleichspannung versorgt ist und wobei die von den Gruppen abgegebenen Spannungen aufsummiert sind zur Abgabe am Ausgang.

Die Erfindung beruht im Kern auf dem Gedanken, Wechselrichter-Zellen mit unterschiedlicher Gleichspannungsversorgung einzusetzen. Dies bietet den Vorzug, dass mit derselben Anzahl von Wechselrichter-Zellen eine wesentlich größere Anzahl von Spannungsstufen eingestellt werden kann (als mit derselben Anzahl von mit identischer Gleichspannung versorgten Wechselrichter-Zellen eingestellt werden kann), so dass mit derselben Anzahl von Wechselrichter-Zellen eine bessere Kurvenform der ausgegebenen Spannung erreicht wird, und das auch noch mit dem Vorzug der besseren Dynamik verknüpft.

Durch die Aufteilung in diese mindestens zwei Gruppen vereinigt die Erfindung zwei gegensätzlich erscheinende Vorteile, nämlich zum einen eine feine Einstellung auch kleine Spannungen bzw. Spannungsunterschiede mit hoher Geschwindigkeit zu ermöglichen durch das Schalten der Wechselrichter-Zellen der mit niedrigerer Spannung versorgten Gruppe (LVC), und zum anderen schnelle und große Spannungssprünge zu ermöglichen durch das Schalten der Wechselrichter-Zellen der mit höherer Spannung versorgten Gruppe (HVC). Es wird somit durch die Erfindung sozusagen eine zweikanalige Struktur geschaffen, mit einem Schwerlastkanal, in dem die Wechselrichter-Zellen der mit größerer Spannung versorgten Gruppe (HVC) angeordnet sind, und mit einem Dynamikkanal, in dem die Wechselrichter-Zellen der mit niedrigerer Spannung versorgten Gruppe (LVC) angeordnet sind. Durch das häufige und in Bezug auf die Spannungsstufen feinere Takten der Wechselrichter-Zellen der mit niedrigerer Spannung versorgten Gruppe (LVC) kann eine feinere Auflösung auch bei niedrigen Spannungen erreicht werden, während durch das Schalten der Wechselrichter-Zellen der mit höherer Spannung versorgten Gruppe (HVC) schnell auch große Spannungen und/oder große Spannungssprünge eingestellt werden können. Somit wird eine Verbesserung der Dynamik nicht nur Bezug auf zeitliche Veränderung, sondern auch in Bezug auf den Spannungshub und die Spannungshöhe erreicht. Die erfindungsgemäße Anordnung eignet sich somit auch zur Generierung von modulierten Spannungssignalen, wie sie insbesondere für sogenannte Moden bei Elektrochirurgie-Generatoren von Bedeutung sind.

Bevorzugt sind die Wechselrichter-Zellen in mindestens zwei Gruppen aufgeteilt, von denen eine erste Gruppe (LVC) mit niedrigerer Gleichspannung versorgt sind als eine andere, zweite Gruppe (HVC). In diesem Sinn umfasst die erste Gruppe Wechselrichter-Zellen für niedriger Spannung und die zweite Gruppe Wechselrichter-Zellen für höhere Spannung. Dies ermöglicht ein schnelles Einstellen auch von großen Spannungen, indem entsprechend die Wechselrichter-Zellen für höhere Spannung (HVC) betätigt werden. Die Wechselrichter-Zellen für niedrige Spannung (LVC) hingegen bieten den Vorteil, dass wegen ihrer geringeren Versorgungsspannung die Schaltverluste der Wechselrichter-Zellen für niedrige Spannung (LVC) überproportional niedriger sind, und diese somit ein häufiges Schalten deutlich besser tolerieren als die Wechselrichter-Zellen für höhere Spannung (HVC) bzw. dabei auch geringere Schaltverluste entstehen. Sind bspw. die Wechselrichter-Zellen für niedrige Spannung (LVC) auf eine Spannung von lediglich einem Viertel der Wechselrichter-Zellen mit höherer Spannung ausgelegt (bspw. auf 12 V für LVC anstelle von 48 V für HVC), so betragen die Schaltverluste der Wechselrichter-Zellen für niedrige Spannung (LVC) nur ein Sechzehntel der Schaltverluste von den Wechselrichter-Zellen für höhere Spannung (HVC). Damit eignen sich die Wechselrichter-Zellen für niedrige Spannung (LVC) in besonderer Weise nicht nur zur Feineinstellung der auszugebenden Spannung sondern dies kann auch auf ausgesprochen schnelle Weise geschehen. - Es versteht sich, dass auch weitere Gruppen vorgesehen sein können, deren Wechselrichter-Zellen vorzugsweise mit weiteren nochmals anderen Gleichspannungen versorgt sind.

Vorzugsweise umfassen die Gruppen eine erste Gruppe, deren Wechselrichter-Zellen mit niedrigerer Gleichspannung (LVC) versorgt sind als die Wechselrichter-Zellen (HVC) einer anderen, zweiten Gruppe.

Nachfolgend seien einige verwendete Begriffe erläutert:

Im Gebiet der Elektrochirurgie-Generatoren werden unter "hochfrequent" Frequenzen typischerweise im Bereich von 200 kHz bis 4000 kHz verstanden. Optional kann bei vorteilhaften Ausführungsformen auch der Ultraschallbereich abgedeckt sein. Unter Ultraschall-Bereich wird ein Frequenzbereich zwischen 20 kHz und 200 kHz verstanden.

Unter "Hochspannung" werden typischerweise Spannungen bis 10kV, vorzugsweise bis 5000 V verstanden.

Die von Elektrochirurgie-Generatoren bereitgestellte Leistung liegt typischerweise im Bereich zwischen 1 und 500 Watt, wobei die Lastimpedanz stark variieren kann und entsprechend sich Ausgangsspannung und Leistungsabgabe ebenso stark und rasch ändern können.

Unter einer "anderen" Gleichspannung wird eine Gleichspannung mit einem anderen Spannungsbetrag verstanden; eine einfache Polaritätsumkehr ist in diesem Sinn keine andere Gleichspannung.

Vorliegend werden die Begriffe des Zuschaltens/Einschaltens bzw. Wegschaltens/Ausschaltens für die Wechselrichter-Zellen synonym verwendet zu Erhöhung der von Wechselrichter-Zellen abgegebenen Spannung um eine Stufe bzw. Verringern um eine Stufe.

Zweckmäßigerweise sind die Wechselrichter-Zellen bipolar ausgeführt und dazu ausgebildet, mindestens drei verschiedene Ausgangsspannungen auszugeben, nämlich positiv, negativ und Null. Auf diese Weise kann mit einfachen Mitteln eine Steigerung der Anzahl der Spannungsstufen und ferner auch eine symmetrische, bipolare Erzeugung der Wechselspannung erreicht werden. Ferner kann durch "Umpolen" der Wechselrichter-Zellen, also indem die Ausgangsspannung von positiv auf negativ umgeschaltet wird bzw. umgekehrt, bei der Ausgangsspannung der jeweiligen Wechselrichter-Zellen auf einen Schlag eine Spannungsänderung um zwei Stufen erfolgen, wenn sie von positiv auf negativ (oder umgekehrt) schaltet.

Mit Vorteil ist weiter vorgesehen, dass zwischen dem Betrag der Spannung, die von den einzelnen Wechselrichter-Zellen höherer Spannung (HVC) erzeugt ist, und dem Betrag der Spannung, die von den einzelnen Wechselrichter-Zellen niedriger Spannung (LVC) erzeugt ist, ein festes Verhältnis besteht. Zweckmäßigerweise kann dies erreicht werden, indem die Gleichspannungsversorgung der beiden Gruppen von Wechselrichter-Zellen zusammenhängend ausgeführt sind. Besonders vorteilhaft ist es dabei, wenn aus der Gleichspannungsquelle für die Wechselrichter-Zellen höherer Spannung (HVC) mittels eines ratiometrischen Spannungswandlers die Spannungsversorgung für die Wechselrichter-Zellen niedrigere Spannung (LVC) erzeugt ist. Zum einen wird auf diese Weise nur eine echte Gleichspannungsquelle benötigt. Zum anderen wird auf diese Weise erreicht, dass die Gleichspannung für die Wechselrichter-Zellen höherer Spannung (HVC) in einem festen Verhältnis steht zu der Gleichspannung damit der Spannungsabgabe für die Wechselrichter-Zellen niedriger Spannung (LVC). Besonders bevorzugt ist es hierbei, wenn das Verhältnis zwischen den Gleichspannungsquellen für die Wechselrichter-Zellen der beiden Gruppen ein ganzzahliges Spannungs-Vielfaches ist. So ergeben mehrere, und zwar eine Anzahl entsprechend dem Spannungsvielfachen, in Reihe geschaltete Wechselrichter-Zellen niedrigerer Spannung (LVC) genau das Spannungsniveau einer Wechselrichter-Zelle für höhere Spannung (HVC). Dies ermöglicht es, bei Spannungsänderungen anstelle von mehreren Wechselrichter-Zellen niedrigerer Spannung (LVC) eine Wechselrichter-Zelle höherer Spannung (HVC) zu schalten (ggf. verbunden mit weiteren Wechselrichter-Zellen niedrigerer Spannung - LVC) und so mit einer geringeren Anzahl von Schaltvorgängen auch größere Spannungssprünge zu bewerkstelligen. Es hat sich bewährt, wenn das ganzzahlige Spannungs-Vielfache mindestens das Vierfache beträgt.

Zweckmäßigerweise ist zur Ansteuerung der Wechselrichter-Zellen beider Gruppen ein Modulator vorgesehen, der dazu ausgebildet ist Schalthäufigkeiten von den Wechselrichter-Zellen hoher Spannung (HVC) zu verringern, indem wahlweise eine Betätigung der Wechselrichter-Zellen hoher Spannung (HVC) ersetzt ist durch eine Betätigung einer Mehrzahl von den Wechselrichter-Zellen niedriger Spannung (LVC). Hiermit macht sich die Erfindung die Erkenntnis zunutze, dass ein Schalten der Wechselrichter-Zellen höherer Spannung (HVC) für diese eine erheblich größere Belastung ist (verglichen mit der Belastung der Wechselrichter-Zellen niedriger Spannung - LVC) und es dabei zu deutlich größeren Schaltverlusten kommt als beim Schalten von Wechselrichter-Zellen niedrigerer Spannung (LVC). Überraschenderweise gilt dies auch dann, wenn dieselbe Spannung (bspw. 48 V) anstatt durch das Schalten einer Wechselrichter-Zelle höherer Spannung) (HVC) bewerkstelligt wird durch das Schalten von vier Wechselrichter-Zellen niedrigerer Spannung (LVC) mit jeweils 12 V. Dieser auf den ersten Blick paradox erscheinende Zusammenhang beruht auf der Erkenntnis, dass infolge des quadratischen Zusammenhangs zwischen den Schaltverlusten und der Spannungshöhe der Gleichstromversorgung der Wechselrichter-Zelle das Schalten der Wechselrichter-Zelle höherer Spannung (HVC) einen überproportionale Verlust mit sich bringt, der durch das Schalten einer größeren Anzahl (nämlich entsprechend dem Spannungsvielfachen) von Wechselrichter-Zellen niedrigerer Spannung (LVC) verringert werden kann.

Vorzugsweise wirkt der Modulator mit einem Stufensteller zusammen, an den das Referenzsignal angelegt ist und der dazu ausgebildet ist, das Referenzsignal in ein Spannungsstufensignal umzusetzen, das an den Modulator angelegt ist. Auf diese Weise wird eine reproduzierbare und determinierte Diskretisierung des Referenzsignals erreicht, welches zur Ansteuerung der Wechselrichter-Zellen des Multilevel-Inverters verwendet wird und zumindest eine Spannungshöhe (und in der Regel auch die Kurvenform) der erzeugten Wechselspannung vorgibt. An sich ist so für jeden Spannungslevel eindeutig bestimmbar, wie viele Wechselrichter-Zellen höherer Spannung (HVC) und Wechselrichter-Zellen niedrigerer Spannung (LVC) zu schalten sind. So entspricht bspw. die Anzahl zu schaltender Wechselrichter-Zellen höherer Spannung (HVC) dem ganzzahligen Anteil einer Division des Spannungslevels durch die Nennspannung der einzelnen Wechselrichter-Zelle höherer Spannung (HVC), und der verbleibende Rest bestimmt die Anzahl der Wechselrichter-Zellen niedrigerer Spannung (LVC) zur Feinanpassung.

Es ist zweckmäßig, wenn der Modulator weiter dazu ausgebildet ist, anhand von mindestens einem vorgebbaren Parameter die Anzahl der zu schaltenden Wechselrichter-Zellen höherer Spannung (HVC) zu variieren und eine weitere Anzahl der zu schaltenden Wechselrichter-Zellen niedriger Spannung (LVC) zu bestimmen sowie diese zur Kompensation gegenläufig zu schalten. Gemäß diesem besonders vorteilhaften Aspekt der Erfindung ist für eine gegebene auszugebende Spannung die Anzahl der einzuschaltenden Wechselrichter-Zellen für hohe Spannung einerseits und Wechselrichter-Zellen für niedrige Spannung andererseits nicht strikt vorbestimmt, sondern ist wahlweise zu variieren. Es wird so mit dem Modulator eine bewusste Mehrdeutigkeit in Bezug auf die Anzahl der (bei gegebener Spannung) zu schaltenden Wechselrichter-Zellen höherer Spannung geschaffen, wodurch dann in der Folge auch die Anzahl der zu schaltenden Wechselrichter-Zellen niedriger Spannung variiert. Für dieses Variieren bei der Umsetzung eines auszugebenden Spannungssignals auf die Anzahl der zu schaltenden Wechselrichter-Zellen höherer Spannung bzw. niedriger Spannung sieht die Erfindung den Modulator vor, der die sich aus einer strikten, determinierten Umsetzung des Soll-Spannungssignals ergebenden Anzahlen für die Wechselrichter-Zellen höherer und niedriger Spannung variiert. Dieses Variieren kann man auch als ein Verzerren der jeweiligen Anzahl ansehen. Der Modulator verzerrt die sich aus einer strikten deterministischen Aufteilung ergebenden Anzahlen von Wechselrichter-Zellen höherer Spannung (HVC) und Wechselrichter-Zellen niedrigerer Spannung (LVC). Man könnte ihn daher auch als einen planvollen Verzerrer der deterministischen Aufteilung bezeichnen.

Eine aus solcher Variierung resultierende Mehrdeutigkeit, die einer üblichen Umsetzung eines Soll-Spannungssignals in typischerweise digitale Ansteuersignale wesensfremd ist, bringt eine Reihe von Vorteilen mit sich:

Durch das Schalten der Wechselrichter-Zellen für höhere Spannung (HVC) kann schnell ein großer Spannungssprung realisiert werden, während mit den Wechselrichter-Zellen für niedrige Spannung (LVC) eine feine Anpassung an den durch das Referenzsignal bestimmten Kurvenverlauf erfolgen kann. Die genaue Anpassung bedingt ein häufiges Schalten der Wechselrichter-Zellen, allerdings genügt dazu meist das Schalten der Wechselrichter-Zellen niedriger Spannung (LVC), deren Betätigung nur wenig Schaltverluste mit sich bringt. Das in punkto Schaltverluste deutliche aufwendigere Schalten von Wechselrichter-Zellen höherer Spannung (HVC) kann durch den Modulator in seiner Häufigkeit verringert werden. Ein Beispiel hierfür ist eine Schaltregel nach dem Muster, wenn eine HVC-Zelle einmal angeschaltet ist dann bleibt sie auch so lange an wie möglich. So können auf effiziente Weise die Schaltverluste verringert werden, wodurch die Schaltgeschwindigkeit und damit generell die Dynamik erhöht werden kann. Es versteht sich, dass hierbei die Freiheit der Variation durch den Modulator zunimmt, je mehr redundante Wechselrichter-Zellen vorhanden sind, insbesondere Wechselrichter-Zellen mit niedriger Spannung (LVC). Je mehr von diesen vorhanden sind, desto länger kann bei Spannungsveränderungen ein Schalten der Wechselrichter-Zellen höherer Spannung (HVC) hinausgezögert oder vermieden werden. Zweckmä-ßig ist es daher, redundante Wechselrichter-Zellen mit niedriger Spannung (LVC) vorzusehen, also mehr als an sich durch das Verhältnis der hohen zur niedrigen Spannung bestimmt.

Beträgt beispielsweise die Spannung der Wechselrichter-Zellen mit höherer Spannung (HVC) 48 V und die Spannung der Wechselrichter-Zellen niedriger Spannung (LVC) 12 V, so kann anstelle des Einschaltens von einer der Wechselrichter-Zellen höherer Spannung (HVC) dieselbe Spannungssteigerung erreicht werden durch Einschalten von vier der Wechselrichter-Zellen niedriger Spannung (LVC). Beträgt die Spannungssteigerung sogar 60 V, so kann auch dann ein Schalten der Wechselrichter-Zelle höherer Spannung (HVC) vermieden werden, wenn mindestens eine redundante (fünfte) Wechselrichter-Zelle niedriger Spannung (LVC) zur Verfügung steht, die dazu eingeschaltet werden kann. Umgekehrt gilt dies entsprechend bei negativen Spannungen. - Ferner kann dies auch entsprechend gelten in solchen Fällen, wenn statt einer Spannungssteigerung eine Spannungsverringerung ansteht. Wird beispielsweise eine Spannung von 72 V ausgegeben und sind dazu eine Wechselrichter-Zelle höherer Spannung (HVC) sowie zwei Wechselrichter-Zellen niedriger Spannung (LVC) eingeschaltet, so kann für eine Verringerung der Spannung um 36 V ein Schalten (d. h. ein Abschalten der bereits eingeschalteten) Wechselrichter-Zelle höherer Spannung (HVC) vermieden werden, indem zwei der eingeschalteten Wechselrichter-Zellen niedriger Spannung (LVC) abgeschaltet werden und eine dritte mit negativer Spannung von 12 V betrieben wird, woraus sich dann insgesamt eine Spannungsreduktion von den gewünschten 36 V ergibt, und zwar ohne dass dazu die (höhere Schaltverluste aufweisende) Wechselrichter-Zelle höherer Spannung (HVC) geschaltet zu werden braucht. Dies gilt auch dann, wenn die Verringerung der Spannung um 36 V erfolgt Stufe für Stufe. Bei jeder Änderung der Spannungsstufe entscheidet der Modulator, ob die Änderung durch Schalten einer LVC oder durch Schalter einer HVC und, in diesem Beispiel, drei LVCs erfolgen soll.

Mit Vorteil ist weiter vorgesehen, dass der Modulator über ein Freigabesignal betätigt ist, und ein Veränderungsdetektor vorgesehen ist, der dazu ausgebildet ist eine Veränderung des Referenzsignals zu erkennen und das Freigabesignal an den Modulator anzulegen. Somit braucht der Modulator bei unverändertem Referenzsignal keine Umschaltungen vorzunehmen, sondern diese erfolgen lediglich dann, wenn sich das Referenzsignal ohnehin ändert. Letzteres wird durch den Veränderungsdetektor erkannt. Dieser wirkt so mit dem Modulator zusammen, dass er bei erkannter Veränderung ein Freigabesignal an den Modulator anlegt. Auf diese Weise reduziert sich die Tätigkeit des Modulators auf solche Fälle, bei denen eine Veränderung des Referenzsignals auftritt. Es kann aber alternativ oder zusätzlich vorgesehen sein, dass der Veränderungsdetektor das von einem Stufensteller erzeugte (Spannungs-)Stufensignal überwacht. Damit reduziert sich die Aktivität des Modulators auf solche Fälle, wenn die Veränderung des Referenzsignals derart ist, dass sie auch zu einer Änderung des (Spannungs-)Stufensignals führt.

Zweckmäßigerweise weisen die Wechselrichter-Zellen ausgangsseitig jeweils eine Potentialentkopplung auf. Hierbei macht sich die Erfindung zunutze, dass ausgangsseitig der Wechselrichter-Zellen definitionsgemäß Wechselspannung anliegt, so dass mit simplen und kostengünstigen Übertragern auf wenig aufwändige Weise (verglichen mit isolierten Einzel-Gleichspannungsquellen, wie sie eingangsseitig erforderlich wären) eine zuverlässige potenzialmäßige Trennung der von den Wechselrichter-Zellen schließlich abgegebenen Spannungen erreicht werden kann.

Vorzugsweise ist weiter vorgesehen, dass der vorgebbare Parameter eine Schalthäufigkeit der Wechselrichter-Zellen höherer Spannung (HVC) umfasst, und der Modulator ausgebildet ist zur Minimierung dieser Schalthäufigkeit. Weiter kann berücksichtigt werden, dass wegen der höheren Spannung der Wechselrichter-Zellen (HVC) die durch das Schalten bedingte Verlustleistung überproportional größer ist. Es besteht sogar ein quadratischer Zusammenhang, so dass das Schalten einer der Wechselrichter-Zellen höherer Spannung (HVC) das 16-fache an Schaltverlustleistung bewirkt gegenüber dem Schalten einer der Wechselrichter-Zellen niedriger Spannung (LVC) bei einem Spannungsverhältnis von 4 zu 1, wie es bspw. bei 48 V zu 12 V der Fall ist. Indem die Häufigkeit des Schaltens der Wechselrichter-Zellen höherer Spannung (HVC) reduziert wird, kann die Verlustleistung insgesamt beträchtlich reduziert werden. Zweckmäßigerweise ist vorgesehen, dass der vorgebbare Parameter ein Maß für eine Verlustleistung der Wechselrichter-Zellen umfasst, und der Modulator ausgebildet ist durch Betätigung der Wechselrichter-Zellen höherer Spannung (HVC) verursachte Verlustleistung anzugleichen an die durch Betätigung der Wechselrichter-Zellen niedriger Spannung (LVC) verursachte Verlustleistung. Das ist nicht nur günstig für den Wirkungsgrad, sondern bringt auch erhebliche Vorteile in Bezug auf thermische Entlastung der Schaltelemente in den Wechselrichter-Zellen.

Zweckmäßigerweise ist weiter vorgesehen, dass in dem Modulator mindestens zwei alternative Schaltregeln für Spannungsänderungen implementiert sind, die beide zu derselben Spannungsänderung führen aber eine unterschiedliche Anzahl von HVC schalten. Der Modulator kann bei Spannungsänderungen eine Auswahl aus diesen Schaltregeln treffen. In dem diese Schaltregeln vorgegeben sind, kann durch die Schaltregeln ein entsprechendes Verhalten des Modulator programmiert sein. Für eine Spannungssteigerung von beispielsweise 12 V ist ein Beispiel für zwei solche alternativen Schaltregeln "a)" und "b)", dass gemäß Schaltregel a) der Ausgangswert der Wechselrichter-Zellen niedriger Spannung (LVC) um eine Stufe von 12 V erhöht wird und bei den Wechselrichter-Zellen höherer Spannung (HVC) keine Änderung erfolgt; oder alternativ gemäß Schaltregel b) die von den Wechselrichter-Zellen niedriger Spannung (LVC) abgegebene Spannung um drei Stufen mit insgesamt 36 V reduziert wird und gleichzeitig die Ausgangsspannung der Wechselrichter-Zellen höherer Spannung (HVC) um eine Stufe, also um 48 V erhöht wird - im Ergebnis bedeutet dies die gewünschte Steigerung um 12 V. Offensichtlich ist Schaltregel b) die aufwändigere, da bei ihr durch das Schalten einer der Wechselrichter-Zellen höherer Spannung (HVC) wesentlich höhere Schaltverluste auftreten als bei Schaltregel a). Um dies zu erreichen sind vorzugsweise die Schaltregeln so implementiert, dass bei einer Spannungserhöhung gemäß einer der alternativen Schaltregeln die Anzahl der Wechselrichter-Zellen höherer Spannung (HVC) gleich bleibt und eine der Wechselrichter-Zellen niedriger Spannung (LVC) zugeschaltet wird (Schaltregel a), oder gemäß der anderen (Schaltregel b) der alternativen Schaltregeln die Anzahl der geschalteten Wechselrichter-Zellen höherer Spannung (HVC) um eine Eins erhöht und eine Mehrzahl von Wechselrichter-Zellen niedriger Spannung (LVC) gegenläufig schaltet, wobei diese Mehrzahl dem um Eins verminderten Spannungs-Vielfachen entspricht. Dies gilt bei einer Spannungssteigerung um eine Stufe entsprechend eine Abstufung in der ausgebbaren Spannungen der Wechselrichter-Zellen niedriger Spannung (LVC). Für größere Spannungsänderungen mit mehreren solchen Stufen gilt das in Schaltregeln implementierte Schema entsprechend.

Entsprechend umgekehrt gilt dies für den Fall einer Spannungsverringerung. Hierbei ist vorzugsweise vorgesehen, dass gemäß einer der alternativen Schaltregeln die Anzahl der Wechselrichter-Zellen höherer Spannung (HVC) gleich bleibt und eine der Wechselrichter-Zellen niedriger Spannung (LVC) abgeschaltet wird, oder gemäß der anderen der alternativen Schaltregeln die Anzahl der geschalteten Wechselrichter-Zellen höherer Spannung (HVC) um Eins verringert und eine Mehrzahl von Wechselrichter-Zellen niedriger Spannung (LVC) gegenläufig schaltet, wobei diese Mehrzahl dem um Eins verminderten Spannungs-Vielfachen entspricht. Wie schon vorstehend zur Spannungssteigerung erläutert, gilt dies für größere Spannungsänderungen mit mehreren Stufen entsprechend.

Zweckmäßigerweise sind den Schaltregeln jeweils Schaltbereiche zugewiesen sind, wobei die Schaltbereiche vorzugsweise für positive und negative Polarität der Ausgangsspannung verschieden sind. Damit kann auf die Polarität der von dem Inverter abgegebenen Spannung Rücksicht genommen werden. Schaltbereiche definieren Wertebereiche für die Schaltregeln. Zweckmäßigerweise ist vorgesehen, dass bei positiver Ausgangsspannung eine Erhöhung bei den Wechselrichter-Zellen höherer Spannung (HVC) so lange wie möglich verzögert werden soll. Bei einem negativen Wert für die Ausgangsspannung, der sich langsam erhöht in Richtung Nullwert, ist es hingegen zweckmäßig, bei den Wechselrichter-Zellen höherer Spannung (HVC) sobald wie möglich eine Erhöhung zu schalten, ggf. unter Berücksichtigung von Hysterese. Bei einem negativen Wert für die Ausgangsspannung, der sich weiter vom Nullwert entfernt ist es hingegen zweckmäßig, ein Schalten der so lange wie möglich hinauszuschieben und stattdessen die Wechselrichter-Zellen niedriger Spannung (LVC) zu schalten. Auf diese Weise wird erreicht, dass die Wechselrichter-Zellen höherer Spannung (HVC), sobald die Spannung wieder in Richtung Null geht, so früh wie möglich wieder ausschalten. Somit kann der Gefahr einer Sättigung von den Übertragern der Wechselrichter-Zellen höherer Spannung (HVC) wirksam begegnet werden.

Vorzugsweise ist optional eine Hysterese vorgesehen, um ein unnötig häufiges An- und Abschalten von Wechselrichter-Zellen höherer Spannung (HVC) bei geringen Spannungsänderungen zu vermeiden. Besonders zweckmäßig ist es, bei mehrfachem Wechsel zwischen Spannungserhöhung und -verringerung ein Schalten der Wechselrichter-Zellen höherer Spannung (HVC) zu blockieren, wobei vorzugsweise zusätzliche Wechselrichter-Zellen niedriger Spannung (LVC) geschaltet werden, wenn die Spannungserhöhung oder -verringerung den Spannungswert der Wechselrichter-Zellen höherer Spannung (HVC) überschreitet.

Mit Vorteil sind Grenzen der Schaltbereiche dynamisch im Betrieb veränderlich, vorzugsweise in Abhängigkeit von Zustandsgrößen der Wechselrichter-Zellen höherer Spannung (HVC) und niedriger Spannung (LVC), insbesondere deren jeweiliger Einschaltzeit, magnetischem Fluss und/oder Temperatur. Auf diese Weise kann der Betriebszustand der Wechselrichter-Zellen und ihrer Komponenten berücksichtigt werden. Beispielsweise kann der magnetische Fluss in den Wechselrichter-Zellen überwacht werden, um insbesondere eine Sättigung der Wechselrichter-Zellen zu vermeiden. Dies kann in der Weise geschehen, dass ein Schaltzustand für die Abgabe positiver Spannung so gewählt wird, dass er genauso lang ist wie der Schaltzustand für die Abgabe negativer Spannung. Auf diese Weise kann ein Ausgleich erzielt werden. Dies ist von besonderer Bedeutung für die Wechselrichter-Zellen höherer Spannung (HVC), da sie definitionsgemäß mit höherer Spannung gespeist werden und seltener geschaltet werden (sollen), was die Gefahr einer ungleichmäßigen Belastung erhöht.

Optional kann eine Überwachungseinheit vorgesehen sein, die dazu ausgebildet ist magnetischen Fluss in den Wechselrichter-Zellen höherer Spannung (HVC) und/oder Wechselrichter-Zellen niedriger Spannung (LVC) zu ermitteln und einen so ermittelten Wert für den magnetischen Fluss zu speichern. Somit kann der magnetische Fluss in den jeweiligen Wechselrichter-Zellen überwacht und eine Sättigung der Wechselrichter-Zellen vermieden werden, in dem sie dann bevorzugt mit entgegengesetzter Spannungspolarität geschaltet ist, bis der magnetische Fluss wieder ausgeglichen ist. Mit Vorteil kann eine Ausgleichseinheit vorgesehen sein, die mit der Überwachungseinheit zusammenwirkt und so ausgebildet ist, dass sie bei einer Spannungserhöhung von den Wechselrichter-Zellen höherer Spannung (HVC) oder Wechselrichter-Zellen niedriger Spannung (LVC) zuerst solche mit niedrigem magnetischen Fluss schaltet, und bei einer Spannungsverringerung zuerst solche mit hohem magnetischen Fluss schaltet. Somit kann auf effiziente Weise ein Ausgleich durchgeführt werden, wodurch eine Überlastung einzelner Wechselrichter-Zellen und das damit einhergehende Ausfallrisiko wirksam verhindert werden kann.

Dazu kann mit Vorteil weiter ein Einschaltzeitmonitor für die Wechselrichter-Zellen höherer Spannung (HVC) oder Wechselrichter-Zellen niedriger Spannung (LVC) vorgesehen sein, der die Zeitdauer einer positiven oder negativen Spannungsabgabe der HVC und LVC ermittelt, und bei Überschreiten eines jeweiligen voreinstellbaren Grenzwerts die jeweilige Wechselrichter-Zellen (HVC oder LVC) abschaltet. Auf diese Weise kann die Verweilzeit der einzelnen Wechselrichter-Zellen wirksam kontrolliert und begrenzt werden. Ist eine der Wechselrichter-Zellen, und relevant sind hierbei insbesondere die Wechselrichter-Zellen höherer Spannung (HVC), zu lange eingeschaltet, so wird sie nicht verwendet sondern in Leerlauf geschaltet und es wird stattdessen eine andere, weniger belastete der Wechselrichter-Zellen höherer Spannung (HVC) betätigt.

Vorzugsweise ist ein Steuersignalgenerator für den Multilevel-Inverter vorgesehen, der dazu ausgebildet ist ein Referenzsignal für die Ansteuerung des Multilevel-Inverters zu erzeugen, wobei das Referenzsignal ein Muster für von dem Elektrochirurgie-Generator abzugebende Wechselspannung ist, insbesondere hinsichtlich Amplitude, Frequenz, Kurvenform und/oder Tastverhältnis, wobei vorzugsweise die Kurvenform frei wählbar einstellbar ist. Mit einem solchen Referenzsignal kann eine präzise Vorgabe an den Multilevel-Inverter in Bezug auf die zu erzeugende Spannung und den Spannungsverlauf erfolgen. Damit ist eine positive Kontrolle über Amplitude sowie Kurvenform der abgegebenen Spannung ermöglicht.

Zweckmäßigerweise ist ein Spitzendetektor vorgesehen, an den das Referenzsignal angelegt ist und das mit seinem Ausgang auf den Modulator wirkt, insbesondere ein Schalten von Wechselrichter-Zellen hoher Spannung (HVC) verringert oder verhindert. Mit Vorteil geschieht in der Weise, dass mit dem Referenzsignal Informationen über den in der Zukunft zu erwartenden Wert für die Ausgangsspannung des Multilevel-Inverters zur Verfügung stehen. Dieses in die Zukunft gerichtete Informationssignal kann von dem Spitzendetektor ausgewertet werden und zur verbesserten Ansteuerung der Wechselrichter-Zellen genutzt werden. Ergibt sich bspw. aus einem Referenzsignal zu einem bestimmten Zeitpunkt, dass die Spannungssteigerung weitgehend abgeschlossen ist und demnächst die Sinuswelle nach Erreichen ihres Maximums wieder zurückgehen wird, so kann für die letzten Schritte zur Spannungserhöhung der Spitzendetektor unter Berücksichtigung des bald auftretenden Spannungsabfalls das Zuschalten eines weiteren Wechselrichter-Zelle für höhere Spannung (HVC) blockiert werden. Stattdessen kann eine weitere der (verlustarm zu schaltenden) Wechselrichter-Zellen niedriger Spannung (LVC) betätigt werden. Dies hat den Vorteil, dass kurz vor dem Erreichen des Maximums unnötige Schaltvorgänge insbesondere der Wechselrichter-Zellen höherer Spannung (HVC) wirksam vermieden werden kann. Stattdessen wird zum Erreichen der Spitzenspannung eine zur Verfügung stehende (redundante) Wechselrichter-Zelle niedriger Spannung (LVC) herangezogen.

Die Erfindung wird nachfolgend näher unter Bezugnahme auf eine vorteilhafte Ausführungsform beispielhaft erläutert. Es zeigen:

Fig. 1　　　eine schematische Darstellung eines Elektrochirurgie-Generators gemäß einem Ausführungsbeispiel mit einem angeschlossen elektrochirurgischen Instrument;

Fig. 2a, b　Blockdiagramme zu Ausführungsbeispielen für einen Multilevel-Inverter des Elektrochirurgie-Generators gemäß Fig. 1 mit kaskadierten Wechselrichter-Zellen;

Fig. 3　　　einen schematisierten Schaltplan von zwei der Wechselrichter-Zellen;

Fig. 4a, b　Blockdiagramme zu Beispielen für einen Selektor mit einem Modulator zu Ansteuerung von Wechselrichter-Zellen hoher und niedriger Spannung;

Fig. 5　　　ein vereinfachtes Beispiel für das Schalten von Wechselrichter-Zellen höherer Spannung und niedriger Spannung zur Umsetzung eines Referenzsignals nach Spannungsstufung;

Fig. 6　　　ein anderes, aufwändigeres Beispiel für das schalten von Wechselrichter-Zellen höherer Spannung und niedriger Spannung zur Umsetzung eines Referenzsignals;

Fig. 7　　　eine Tabelle mit Schaltregeln für den Modulator abhängig von einer Polarität der Ausgangsspannung und einem Ansteigen bzw. Abfallen des Referenzsignals;

Fig. 8　　　eine Tabelle mit veränderlichen Schaltregeln für den Modulator als Variante zu Fig. 7; und

Fig. 9a, b　beispielhafte Schaltverläufe für WechselrichterZellen hoher niedriger Spannung, ohne und mit Berücksichtigung von magnetischer Sättigung in den Wechselrichter-Zellen.

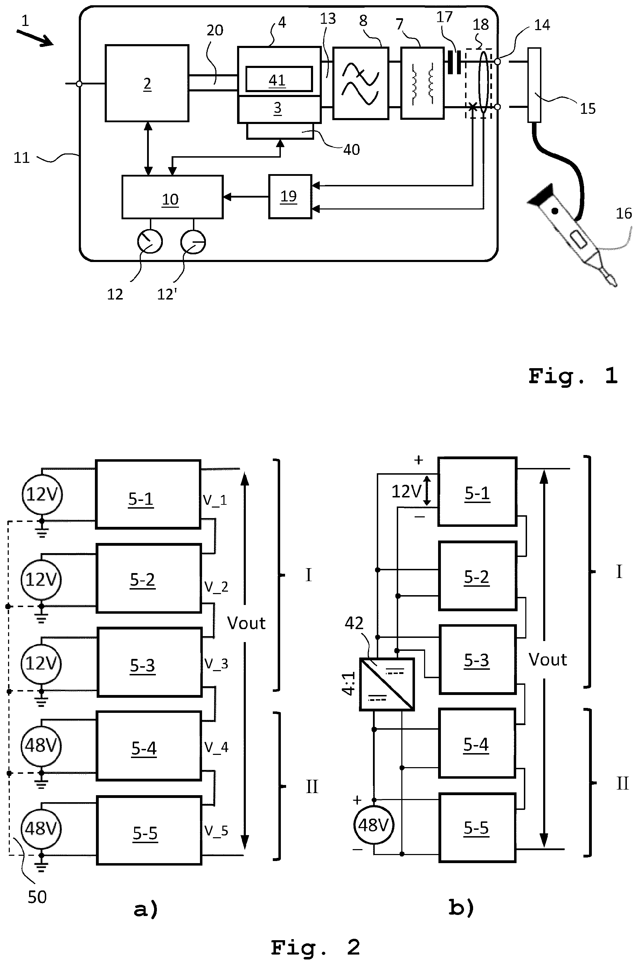

Ein Elektrochirurgie-Generator gemäß einem Ausführungsbeispiel der Erfindung ist in Figur 1 dargestellt. Der in seiner Gesamtheit mit der Bezugsziffer 1 bezeichnete Elektrochirurgie-Generator umfasst ein Gehäuse 11, das mit einem Ausgangsanschluss 14 für ein elektrochirurgisches Instrument 16 versehen ist, in dem dargestellten Ausführungsbeispiel handelt es sich hierbei um ein elektrisches Skalpell. Es ist über einen Anschlussstecker 15 eines Hochvoltverbindungskabels mit dem Ausgangsanschluss 14 des Elektrochirurgie-Generators 1 verbunden.

Die Leistungsabgabe an das elektrochirurgische Instrument 16 kann über einen Leistungssteller 12 verändert werden.

Zur Leistungsversorgung des Elektrochirurgie-Generators 1 ist eine Gleichspannungsversorgung 2 vorgesehen, welche über ein Netzanschlusskabel (nicht dargestellt) mit dem öffentlichen Stromnetz verbunden werden kann und daraus gespeist ist. Bei der Gleichspannungsversorgung 2 handelt es sich bei dem dargestellten Ausführungsbeispiel um ein Netzteil. Es umfasst einen Gleichrichter und speist einen Gleichspannungszwischenkreis 20 mit Gleichspannung, deren Höhe vorzugsweise fest ist und beispielsweise 48 Volt beträgt. Es soll aber nicht ausgeschlossen sein, dass die Gleichspannungshöhe variabel ist zwischen 0 und etwa 400 Volt, wobei die absolute Höhe der Gleichspannung insbesondere abhängen kann von der eingestellten Leistung, der Art des elektrochirurgischen Instruments 16 und/oder dessen Lastimpedanz, die wiederum abhängt von der Art des behandelten Gewebes. Notwendig ist ein internes Netzteil jedoch nicht, so kann die Gleichspannungsversorgung auch durch ein externes Netzteil realisiert sein, oder es ist eine direkte DC-Speisung vorgesehen, bspw. 24 Volt in Fahrzeugen oder 48 Volt bei stationären Anwendungen.

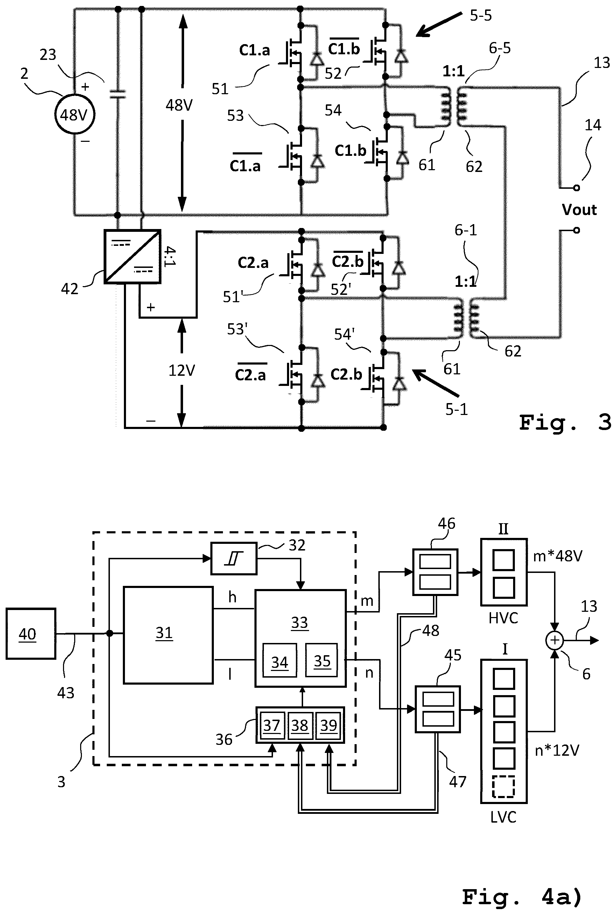

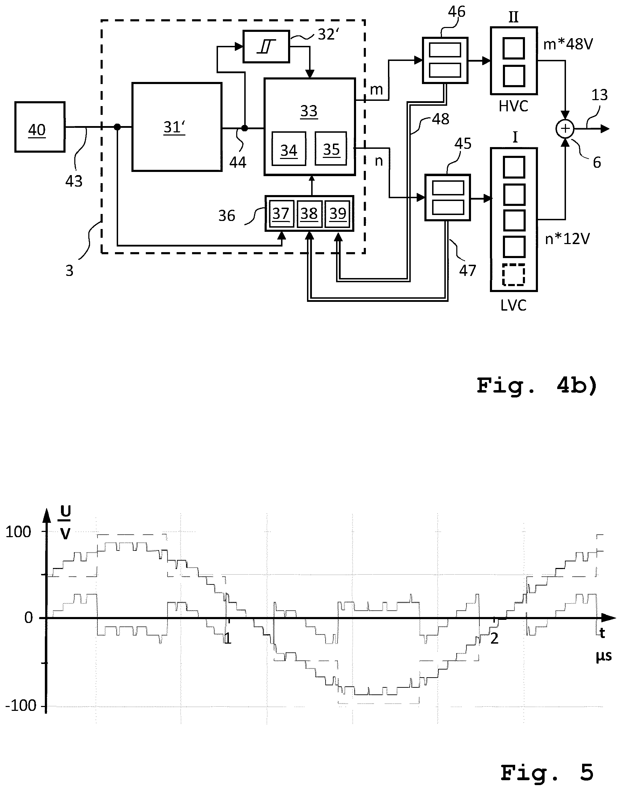

Von dem Gleichspannungszwischenkreis 20 gespeist ist ein Inverter, der aus zugeführter Gleichspannung hochfrequente Wechselspannung im Hochspannungsbereich erzeugt, mit vorgebbaren Frequenzen im Bereich zwischen 200 kHz und 4 MHz. Der Inverter ist in der Bauart als Multilevel-Inverter 4 ausgeführt, wie nachfolgend noch näher erläutert werden wird. Frequenz und Kurvenform der vom Multilevel-Inverter 4 zu erzeugenden hochfrequenten Wechselspannung sind hierbei vorgegeben von einer Invertersteuerung 41 auf Basis eines von einem Steuersignalgenerator 40 erzeugten Referenzsignals 43 (s. Fig. 4). Die vom Multilevel-Inverter 4 erzeugte hochfrequente Wechselspannung wird über eine Abgabeleitung 13, einen Ausgangstransformator 7 zum Hochsetzen der Ausgangsspannung in den Bereich von einigen Kilovolt sowie einen Tiefpass 8 geführt und, abgesichert durch einen Blockkondensator 17 gegen unerwünschte Gleichstromanteile, am Ausgangsanschluss 14 ausgegeben zum Anschluss für das elektrochirurgische Instrument 16. Ferner werden Spannung und Strom der vom Multilevel-Inverter 4 erzeugten und ausgegebenen Hochspannung gemessen mittels eines kombinierten Spannung- und Stromsensors 18 und die Messsignale werden einer Verarbeitungseinheit 19 zugeführt, welche die entsprechenden Daten über die abgegebene Spannung, Strom und Leistung als Rückführung an eine Betriebssteuerung 10 des Elektrochirurgie-Generators 1 anlegt, die ihrerseits mit dem Steuersignalgenerator 40 kommuniziert. An die Betriebssteuerung 10 ist auch der Leistungssteller 12 angeschlossen. Die Betriebssteuerung 10 ist ferner dazu ausgebildet, verschiedene sog. Moden einzustellen, bei denen es sich typischerweise um eingespeicherte Spannungs-/Zeitverläufe handelt. Zur Auswahl des Modus durch den Nutzer ist ein Auswahlschalter 12' vorgesehen. Die Betriebssteuerung 10 wirkt ferner zusammen mit dem Steuersignalgenerator 40, der dazu ausgebildet ist das Referenzsignal 43 für die auszugebende Wechselspannung, insbesondere in Bezug auf Amplitude, Frequenz, Kurvenform und Tastverhältnis zu erzeugen und an eine Invertersteuerung 41 auszugeben.

Der Multilevel-Inverter 4 umfasst eine Mehrzahl von in Reihe geschalteten Wechselrichter-Zellen 5, die angesteuert sind von der Invertersteuerung 41. Die Wechselrichter-Zellen 5 sind in zwei Gruppen I und II unterteilt, die gruppenweise mit Gleichspannung verschiedener Höhe gespeist sind. Eine erste ist als "Gruppe I" bezeichnet und umfasst Wechselrichter-Zellen niedriger Spannung (LVC), und zwar bei dem Beispiel in Figur 2 drei Wechselrichter-Zellen 5-1, 5-2 bis 5-3. Sie sind gespeist von einer Gleichspannungsquelle mit niedriger Gleichspannung, im Beispiel 12 V. Ferner ist eine Gruppe von Wechselrichter-Zellen hoher Spannung (HVC) gebildet, die als "Gruppe II" bezeichnet ist und in dem in Figur 2 dargestellten Beispiel zwei Wechselrichtern-Zellen 5-4, 5-5 umfasst, die mit einer höheren Gleichspannung, im Beispiel 48 V, gespeist sind. Die von den beiden Gruppen I und II ausgegebenen Spannungen werden mittels Übertrager 6 summiert (siehe Figur 4a, b). Ferner ist ein Selektor 3 vorgesehen, der die anzusteuernden Wechselrichter-Zellen 5 bestimmt, insbesondere die Anzahl "m" der anzusteuernden Wechselrichter-Zellen hoher Spannung (HVC) der Gruppe II und die Anzahl "n" der anzustellenden Wechselrichter-Zellen niedriger Spannung (LVC) der Gruppe I.

Es wird nun Bezug genommen auf Figur 2a. Bei dem dort dargestellten Ausführungsbeispiel ist an dem Eingang (in der Zeichnung linksseitig dargestellt) jeder der Wechselrichter-Zellen 5 eine Gleichspannungsquelle mit einer bestimmten Gleichspannung angeschlossen. Die jeweilige Wechselrichter-Zelle 5 erzeugt daraus eine Wechselspannung, die an dem Ausgang (in der Zeichnung rechtsseitig dargestellt) der jeweiligen Wechselrichter-Zelle 5 als Wechselspannung abgegeben wird. Die Anzahl der Wechselrichter-Zellen ist nicht beschränkt und an sich beliebig. Die Wechselrichter-Zellen 5 sind in der Figur 2a durchnummeriert mit der Bezeichnung "5-1", "5-2" bis "5-5", wobei die Anzahl 5 beispielhaft ist und eine beliebige Anzahl von mindestens zwei Wechselrichter-Zellen vorgesehen sein kann. Die am Eingang der jeweiligen Wechselrichter-Zelle 5 angelegten Gleichspannungen sind optional potenzialmäßig gekoppelt über eine Leitschiene 50. Entsprechend ist die am Ausgang der jeweiligen Wechselrichter-Zelle 5 ausgegebene Wechselspannung bezeichnet als "V_1", "V_2" bis hin zu "V_5". Durch die Reihenschaltung der Wechselrichter-Zellen 5 erfolgt eine Addition von deren Ausgangsspannungen, sodass schließlich sich als Gesamt-Ausgangsspannung ergibt:

$$V_{out} = \sum_{i=1}^{N} V\_i$$

Verglichen mit einer einfachen Bauweise mit nur einer Gleichspannungsquelle erhöht sich zwar erfindungsgemäß der Aufwand in Bezug auf Gleichspannungsquellen, weil nunmehr mehrere (im Beispiel: zwei) statt nur einer benötigt werden. Aber dafür erhöht sich die Zahl der Spannungsstufen beträchtlich, und zwar ausgehend von elf Spannungsstufen bei nur einer Gleichspannungsquelle auf mehr als das Doppelte mit 23 Spannungsstufen. Die Anzahl der so erreichbaren Spannungsstufen folgt der Formel

$$2 * (mHVc * r + nLVc) + 1 \quad ,$$

wobei mHVc für die Anzahl der Wechselrichter-Zellen mit höherer Gleichspannung (in obigem Beispiel m=2), nLVc für die Anzahl der Wechselrichter-Zellen mit niedriger Gleichspannung (in obigem Beispiel n=3) und r für das Verhältnis von höherer zu niedriger Gleichspannung (in obigem Beispiel r=4) steht.

Die beiden Gleichspannungsquellen brauchen potenzialmäßig nicht voneinander isoliert zu sein, sondern sie können sich ein gemeinsames Bezugspotential teilen, wie es in Figur 2a realisiert ist mittels der Leitschiene 50. Dies ermöglicht es ferner aus der höheren Gleichspannung, bei der es sich beispielsweise um die Gleichspannung im Zwischenkreis 20 handeln kann, die niedrigere Gleichspannung mittels eines Gleichspannungswandlers 42, insbesondere eines DC/DC-Tiefsetzstellers, zu erzeugen. Im vorliegenden Beispiel wäre er ausgelegt für eine ratiometrische Versorgung mit einem Untersetzungsverhältnis von 4:1, wie es in Figur 2b dargestellt ist. Ein Vorteil dieser Konfiguration mittels ratiometrischer Versorgung ist, dass Änderungen oder Schwankungen in der höheren Gleichspannung sich dann in proportionaler Form widerspiegeln in der niedrigeren Gleichspannung, so dass die relative Stufung erhalten bleibt. Damit kann beispielsweise eine Erhöhung um der Ausgangsspannung um 12V auf zwei verschiedene Weisen erfolgen, einmal konventionell durch Zuschalten einer weiteren 12V-Wechselrichter-Zelle oder durch Zuschalten einer 48V-Wechselrichter-Zellen kombiniert mit Wegschalten von drei 12V-Wechselrichter-Zellen.

Der Aufbau der einzelnen Wechselrichter-Zellen 5 und deren Zusammenwirken sind in dem schematisierten Schaltplan gemäß Figur 3 beispielhaft dargestellt. Insgesamt sind dort zwei Wechselrichter-Zellen 5-1 und 5-5 in kaskadierter Anordnung gezeigt, um so auch deren Versorgung mit Gleichspannung unterschiedlicher Höhe darzustellen. Am linken Bildrand ist die gemeinsame Gleichspannungsquelle 2 dargestellt mit einer Versorgungsspannung Vin von 48 Volt. Ihr zugeordnet ist ein Stabilisierungskondensator 23. Hierdurch werden die beiden Wechselrichter-Zellen 5-3 und 5-4 mit Gleichspannung versorgt. Nachfolgend wird zuerst Bezug genommen auf die Schaltung der Wechselrichter-Zelle 5-5, die mit 48 Volt versorgt ist. Vorgesehen sind vier Leistungsschalter, die als Stromventile fungieren und in H-Brückenschaltung angeordnet sind. Bei den Leistungsschaltern handelt sich um Halbleiterleistungsschalter, beispielsweise ausgeführt als IGBT, MOS-FET, GaN-FET. Die Leistungsschalter 51, 53 sind in Reihe geschaltet und bilden einen ersten Zweig, und die Leistungshalbleiter 52, 54 sind ebenfalls in Reihe geschaltet und bilden einen zweiten Zweig. Die Mittelpunkte der beiden Zweige sind rausgeführt und an die beiden Enden einer Primärwicklung 61 eines ersten Übertragers 6-5 angeschlossen. Der Übertrager 6-5 weist ferner eine Sekundärwicklung 62 auf, wobei das Übersetzungsverhältnis 1:1 beträgt (es sei angemerkt, dass auch ein anderes Übersetzungsverhältnis vorgesehen sein kann, insbesondere um eine Vorverstärkung zu erreichen, bspw. mit einem Übersetzungsverhältnis von 1:2). An die Sekundärwicklung 62 angeschlossen ist eine Abgabeleitung 13, die zum Ausgangsanschluss 14 des Elektrochirurgie-Generators 1 führt (ggf. über ein in Fig. 3 nicht dargestelltes Tiefpassfilter 8 und einen Ausgangstransformator 7, s. Fig. 1).

Die beiden Leistungsschalter 51, 53 des ersten Zweigs sind mit einem gemeinsamen Signal C1.a angesteuert, wobei dem Leistungsschalter 53 dieses Signal invertiert zugeführt ist. Entsprechend sind die beiden Leistungsschalter 52, 54 des zweiten Zweigs ebenfalls mit einem gemeinsamen Signal C1.b angesteuert, wobei dem Leistungsschalter 52 dieses Signal invertiert zugeführt ist. Die Signale C1.a und C1.b sind in an sich bekannter Weise erzeugt von der Invertersteuerung 41. Das bedeutet, dass bei einem HIGH-Signal von C1.a der Leistungsschalter 51 durchschaltet und der Leistungsschalter 53 sperrt, also der erste Leistungszweig ein positives Potenzial an den oberen Anschluss der Primärwicklung 61 des Übertragers 6-1 anlegt. Entsprechend schaltet im zweiten Leistungszweig bei einem HIGH-Signal von C2.b der Leistungsschalter 54 durch, während der Leistungsschalter 52 sperrt. Damit legt der zweite Leistungszweig ein negatives Potenzial an den unteren Anschluss der Primärwicklung 61 an. Bei LOW-Signal von C1.a bzw. C1.b gilt dies entsprechend umgekehrt, d. h. die Polarität an der Primärwicklung 61 kehrt sich um. Somit wird eine Wechselspannung durch die Wechselrichter-Zelle 5-1 erzeugt und an der Primärwicklung 61 des Übertragers 6-1 angelegt.

Die zweite Wechselrichter-Zelle 5-1 weist einen identischen Aufbau auf, ist jedoch aus der Gleichspannungsquelle 2 gespeist über den ratiometrischen Gleichspannungswandler 42, der eine Untersetzung auf ein Viertel der Eingangsspannung bewirkt. Er gibt somit eine Gleichspannung von 12 Volt aus, mit der die zweite Wechselrichter-Zelle 5-1 in an sich gleicher Weise wie die erste Wechselrichter-Zelle 5-5 versorgt wird. In der Figur sind daher für gleichartige Elemente dieselben Bezugsziffern verwendet. Angesteuert wird sie mittels Steuersignalen C2.a und C 2.b, die von der Invertersteuerung 41 bspw. mittels an sich bekannter Pulsweitenmodulation (PWM) erzeugt sind, in entsprechender Weise wie vorstehend beschrieben. Sie gibt somit an ihrem Ausgang ebenfalls eine Wechselspannung aus, die an eine Primärwicklung 61 eines zweiten Übertragers 6-1 angelegt ist. Je nach Ausführung des Gleichspannungswandlers 42 sind die beiden Wechselrichter-Zellen 5-1 und 5-5 potenzialmäßig verbunden. Das bedeutet, dass die von den Wechselrichter-Zellen 5-1 und 5-5 unmittelbar ausgegebenen Wechselspannungen nicht ohne weiteres addiert werden können, da sie bezüglich ihres Potenzials miteinander verknüpft sind. Indem diese ausgegebenen Wechselspannung aber jeweils den Übertragern 6-1 und 6-5 zugeführt werden, sind die von den Übertragern 6-1 und 6-5 ausgegebenen Wechselspannungen jeweils potentialfrei und können ohne weiteres miteinander addiert werden zu einer gemeinsamen Ausgangsspannung, die an der Abgabeleitung 13 anliegt. Sind die Wechselrichter-Zellen 5-1 und 5-5 durch eine entsprechende Gestaltung des Gleichspannungswandlers aber potentialmäßig entkoppelt, so können die ausgegebenen Wechselspannungen auch ohne diese Übertrager unmittelbar durch Reihenschaltung addiert werden.

Die so (und von weiteren Wechselrichter-Zellen 5-2 bis 5-4) erzeugte und summierte Gesamtspannung wird ausgegeben über die Abgabeleitung 13, an deren Ende das Tiefpassfilter 8 angeordnet ist. Es kann bspw. als Filter zweiter Ordnung ausgeführt sein umfassend eine Induktivität sowie eine Kapazität. Es sei angemerkt, dass auch Streu-Induktivitäten der Übertrager 6-1 bis 6-n zu der Induktivität des Tiefpassfilters beitragen und diese ggf. zumindest zum Teil ersetzen können. Das Tiefpassfilter 8 ist so abgestimmt, dass Störungen in der erzeugten Wechselspannung aufgrund der Schaltfrequenz der Leistungsschalter in den Wechselrichter-Zellen 5 des Multilevel-Inverters 4 ausgefiltert werden. Der Ausgang des Tiefpassfilters 8 ist angelegt an eine Primärwicklung des Ausgangstransformators 7, der eine galvanische Trennung des an die Sekundärwicklung angeschlossenen Ausgangsanschluss 14 bewirkt. Ferner ist ein Blockkondensator 17 vorgesehen. Dieser dient als Sicherheitselement dazu, die Abgabe von Gleichstromanteilen an das chirurgische Instrument 16 zu verhindern.

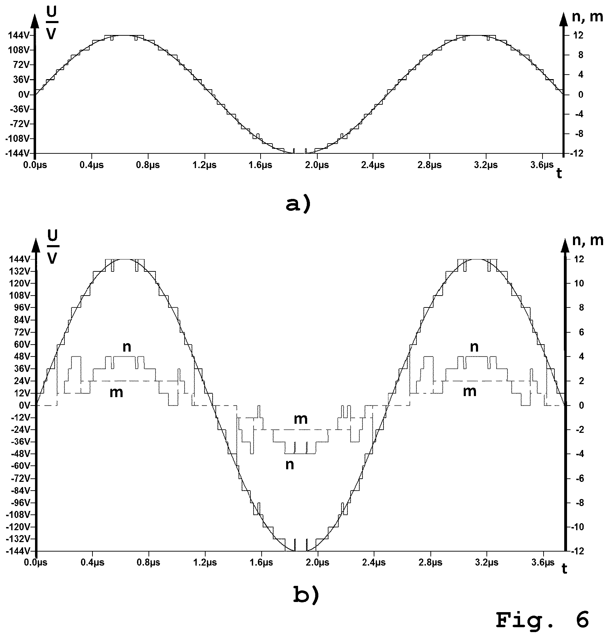

Der Steuersignalgenerator 40 erzeugt insbesondere auf Basis von Vorgaben der Betriebssteuerung 10 ein Referenzsignal 43 für die Ansteuerung des Multilevel-Inverters 4. Hierbei handelt sich um ein Wechselspannungs-Signal, dass typischerweise sinusförmig ist und eine bestimmte Frequenz und Amplitude aufweist. Es wird nun Bezug genommen auf Figur 4a, b. Das Referenzsignal 43 ist angelegt an den Selektor 3, der daraus die Anzahl und den Typ (HVC bzw. LVC) der anzusteuernden Wechselrichter-Zellen 5 bestimmt, und zwar aufgeschlüsselt nach Wechselrichter-Zellen niedriger Spannung (LVC) aus Gruppe I und Wechselrichter-Zellen höherer Spannung (HVC) aus Gruppe II. Dazu umfasst der Selektor 3 einen Stufensteller 31 sowie einen Modulator 33. Der Stufensteller 31 ist dazu ausgebildet, das typischerweise kontinuierliche Referenzsignal 43 umzusetzen in ein Spannungsstufen-Signal. Hierbei handelt sich um ein diskretes Signal, welches indikativ für die Anzahl der Spannungsstufen ist, und zwar typischerweise ausgedrückt in Stufen deren Höhe sich aus der Spannung der Wechselrichter-Zellen niedriger Spannung (LVC) der Gruppe I ergibt, im vorliegenden Beispiel also in Stufen von 12 V. Ein Beispiel für ein solches kontinuierliches Referenzsignal 43 sowie ein daraus gebildetes Stufensignal ausgedrückt in Stufen von 12 V ist in Figur 6a abgebildet. Die stufige Kurve zeigt das Spannungsstufen-Signal und bildet somit eine Diskretisierung des durch die kontinuierliche Kurve dargestellten Referenzsignals 43.

Ferner kann der Stufensteller 31 bereits eine vorläufige Aufteilung dafür vornehmen, welcher Anteil hiervon auf die Wechselrichter-Zellen niedriger Spannung (LVC) der Gruppe I bzw. auf die Wechselrichter-erzählen höherer Spannung (HVC) der Gruppe II entfällt. Dies kann beispielsweise bei einer Ausführungsform, wie sie in Figur 4a dargestellt ist, dadurch geschehen, indem die Grund-Anzahl der zum Erreichen der Spannung gemäß Referenzsignal 43 erforderlichen Wechselrichter-Zellen minimiert ist. Eine solche Aufteilung kann zwei Signale umfassen, ein Signal "h" für die Grundanzahl der einzuschaltenden Wechselrichter-Zellen höherer Spannung (HVC) der Gruppe II und ein Signal "I" für die Grundanzahl der einzuschaltenden Wechselrichter-Zellen niedriger Spannung (LVC) der Gruppe I. Hierbei kann es sich insbesondere um eine rein numerische Aufteilung handeln, bspw. das zum Erzeugen einer Spannung von 84 V genau eine Wechselrichter-Zelle höherer Spannung (HVC) aus Gruppe II und drei Wechselrichter-Zellen niedriger Spannung (LVC) aus Gruppe I anzusteuern sind.

Allerdings werden diese Grundanzahlen "h" und "I" nicht unmittelbar zur Ansteuerung verwendet, sondern sie werden variiert mittels des Modulators 33. Der Modulator 33 ist dazu vorgesehen, die Schalthäufigkeiten der Wechselrichter-Zellen hoher Spannung (HVC) gemäß Gruppe II zu verringern. Ersatzweise werden dafür Wechselrichter-Zellen niedriger Spannung (LVC) gemäß Gruppe I geschaltet. Nachfolgend wird dies näher beschrieben. Die sich so ergebende Aufteilung durch den Modulator 33 in die Anzahlen "n" für die zu schaltenden Wechselrichter-Zellen niedriger Spannung (LVC) der Gruppe I und "m" für die zu schaltenden Wechselrichter-Zellen höherer Spannung (HVC) der Gruppe II ist situativ verschieden und erfindungsgemäß uneindeutig.

Die somit variierte Anzahl "n" für die zu schaltenden Wechselrichter-Zellen niedriger Spannung (LVC) der Gruppe I sowie die variierte Anzahl "m" sind als Ausgangssignale von der Modulator 33 ausgegeben und an Untersteuerungen 45, 46 für die Wechselrichter-Zellen LVC der Gruppe I bzw. HVC der Gruppe II angelegt. Diese steuern in an sich bekannter Weise die jeweiligen Wechselrichter-Zellen LVC in der Gruppe I bzw. HVC in der Gruppe II an. Die Untersteuerungen 45, 46 erfassen Schaltdaten zu den einzelnen Wechselrichter-Zellen 5 der Wechselrichter-Zellen niedriger Spannung (LVC) der Gruppe I bzw. der Wechselrichter-Zellen hoher Spannung (HVC) der Gruppe II. Diese umfassen u.a. Einschaltdauer, Zähler für die Anzahl der Schaltvorgänge sowie den magnetischen Fluss durch die einzelnen Wechselrichter-Zellen 5 und deren Übertrager 6. Sie übermitteln entsprechende Zustandsdaten über Datenleitungen 47, 48 an den Modulator 33 und/oder ein ihm vorgelagertes Adaptionsmodul 36.

Der Modulator 33 braucht nicht zwingend fortdauernd zu arbeiten. Es kann genügen, wenn er insbesondere dann betätigt wird und die Aufteilung des Spannungsstufen-Signals in die Anzahl der zu schaltenden Wechselrichter-Zellen niedriger Spannung (LVC) und hoher Spannung (HVC) durchführt, wenn sich eine Änderung im Spannungsstufen-Signal oder im Referenzsignal 43 ergeben hat. Dazu ist optional ein Veränderungsdetektor 32 vorgesehen, der das Referenzsignal 43 überwacht und bei Veränderung den Modulator 33 betätigt.

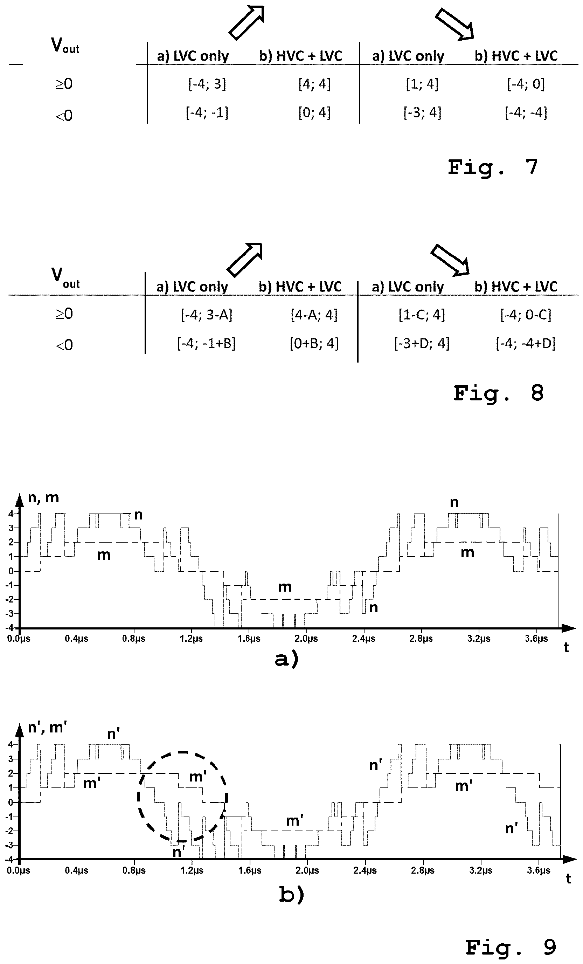

Bei einer alternativen Ausführungsform, wie sie in Figur 4b dargestellt ist, ist der Stufensteller 31' abweichend so ausgeführt, dass er lediglich ein diskretisiertes Referenzsignal (Stufensignal) 44 ausgibt. Daraus bestimmt der Modulator 33 direkt die Anzahl "m" der zu schaltenden Wechselrichter-Zellen hoher Spannung (LVC)HVC und die Anzahl "n" der zu schaltenden Wechselrichter-Zellen niedriger Spannung (LVC). Dies sei anhand eines vereinfachten Beispiels erläutert: Der Stufensteller 31' generiert aus dem Referenzsignal 43 ein diskretes Stufensignal 44. Der Modulator 33 ist dazu ausgebildet, das Stufensignal 44 zu vergleichen mit dem letzten vorherigen Wert des Stufensignals. Der Vergleich kann ergeben, dass ein Anstieg, ein Abfall oder Konstanz vorliegt. Dies wird mittels des Veränderungsdetektors 32' erfasst. Nur in dem Fall eines Anstiegs oder eines Abfalls, also nur dann wenn eine Änderung gegenüber dem vorherigen Stufensignal eingetreten ist, werden die Werte für die Anzahlen "m" und "n" angepasst. Dies erfolgt mittels der Schaltregeln, wie sie weiter unten unter Bezugnahme auf die in Figuren 7 und 8 dargestellten Beispiele erläutert werden.

Ein Beispiel für die Spannungserzeugung durch Wechselrichter-Zellen höherer Spannung (HVC) ist in Figur 5 durch eine gestrichelte Linie dargestellt, und mit der durchgezogenen Linie nahe der Nulllinie ist die von den Wechselrichter-Zellen niedriger Spannung (LVC) erzeugte Spannung dargestellt. Zusammen ergeben sie den gewünschten sinusartigen Verlauf, wie durch die quantisierte Sinuslinie dargestellt ist. Man erkennt, dass jede der beiden Wechselrichter-Zellen höherer Spannung (HVC) der Gruppe II je Halbwelle nur einmal ein- bzw. ausgeschaltet zu werden braucht, und die weitere Anpassung durch häufiges Schalten der Wechselrichter-Zellen niedriger Spannung (LVC) der Gruppe I erfolgt. Hierbei nehmen die LVC der Gruppe I sowohl eine Erhöhung der Spannung vor (bspw. gleich am Anfang im Zeitraum von 0 bis 0,25 ps) wie auch eine Verringerung durch kompensatorisches Gegenschalten zur Verkleinerung der von den Wechselrichter-Zellen HVC abgegebenen, temporär zu hohen Spannung (beispielsweise im Zeitraum 0,25 bis 0,65 ps und 0,87 bis 0,98 ps). Diese Weise können Schaltvorgänge der Wechselrichter-Zellen höherer Spannung (HVC) vermieden und somit deren Anzahl reduziert werden, und damit auch die durch das Schalten der HVC Zellen entstehende erhebliche Schaltverlustleistung reduziert werden.

Ein komplexeres Beispiel für mehr Wechselrichter-Zellen ist in Figur 6b dargestellt. Die sich dabei ergebenden Werte "m" für die Anzahl der einzuschaltenden Wechselrichter-Zellen höherer Spannung (HVC) der Gruppe II sind durch eine gestrichelte Linie und für die Anzahl der einzuschaltenden Wechselrichter-Zellen niedriger Spannung (LVC) der Gruppe I mit durchgezogener gestufter Linie "n". Man erkennt deutlich am Verlauf der mit "m" bezeichneten Linie, dass die Schaltaktivität der Wechselrichter-Zellen höherer Spannung (HVC) beträchtlich verringert ist, insbesondere im Bereich der Amplitudenmaxima des Referenzsignals und des Nulldurchgangs. Indem Schaltaktivitäten der Wechselrichter-Zellen niedriger Spannung (LVC) priorisiert werden, können so die höhere Schaltverluste aufweisenden Wechselrichter-Zellen höherer Spannung (HVC) geschont werden.

Um dies zu erreichen, sind in dem Modulator 33 Schaltregeln 34 implementiert. Den Schaltregeln liegt eine beispielhafte Konfiguration zugrunde mit zwei Wechselrichter-Zellen höherer Spannung (HVC) in Gruppe II und 4 Wechselrichter-Zellen niedriger Spannung in Gruppe I, wie auch in der Figur 4a, b dargestellt. Unter Nutzung des Veränderungsdetektors 32 werden die Schaltzustände der Wechselrichter-Zellen nur dann verändert, wenn sich auch das Referenzsignal 43 verändert hat. Die Schaltregeln 34 sehen für den Fall eines Anstiegs zwei mögliche Alternative vor:

a) Erhöhen der von den Wechselrichter-Zellen niedriger Spannung (LVC) ausgegebenen Spannung um 1 Stufe (entsprechend 12 V) ohne Veränderung betreffend die Wechselrichter-Zellen hoher Spannung (HVC); oder

b) Verringern der von den Wechselrichter-Zellen niedriger Spannung (LVC) ausgegebenen Spannung um 3 Stufen (entsprechend -36 V) und Erhöhen der von den Wechselrichter-Zellen hoher Spannung (HVC) ausgegebenen Spannung um eine Stufe (+48 V).

Beide Alternativen a), b) führen zu derselben Spannungsänderung um eine Stufe, nämlich um +12 V. Alternative b) verlangt ein Schalten von einer der Wechselrichter-Zellen hoher Spannung (HVC), was wegen des quadratischen Zusammenhangs mit der vierfachen Versorgungsspannung auf das 16-fach erhöhte Schaltverluste bedeutet verglichen mit einer der Wechselrichter-Zellen niedriger Spannung (LVC). Hinzu kommen noch drei Schaltvorgänge der Wechselrichter-Zellen niedriger Spannung (LVC). Somit bedeutet die Alternative b) eine 19-fach höhere Verlustenergie als Alternative a) der Schaltregeln 34.

Diesen Zusammenhängen tragen die in Figur 7 dargestellten Schaltregeln 34 Rechnung. Es wird nun Bezug genommen auf die linke Spalte, welche den Fall eines Spannungsanstiegs betrifft. Dort sind die beiden Alternativen a) und b) enthalten. Eingangsparameter sind die Polarität der Ausgangsspannung und die von den Wechselrichter-Zellen niedriger Spannung (LVC) der Gruppe I abgegebene Spannung, ausgedrückt in Spannungsstufen der LVCs. Hierbei steht "1" für eine abgegebene Spannung von +12 V, "4" für eine abgegebene Spannung von +48 V, und entsprechend "-4" für eine abgegebene Spannung von -48 V. Die in dem Modulator 33 implementierten Schaltregeln 34 besagen nun für positive Polarität der Ausgangsspannung, dass bei einer Spannungsstufe zwischen -4 und 3 (entsprechend -48 V bis +36 V) der Gruppe I die Schaltregel a) zur Anwendung kommt, d. h. die von den LVC-Wechselrichter-Zellen der Gruppe I abgegebene Spannung wird um 12 V erhöht. Liegt jedoch bereits Spannungsstufe 4 bei der Gruppe I vor, so kommt die alternative Schaltregel b) zur Anwendung, bei der die HVC-Wechselrichter der Gruppe II um eine Stufe hoch geschaltet werden, wodurch sich eine Erhöhung um 48 V ergibt, und die LVC-Wechselrichter-Zellen der Gruppe I kompensatorisch um drei Stufen runter geschaltet werden, entsprechend -36 V, woraus schließlich die gewünschte Erhöhung um 12 V resultiert. Ist die Polarität der Ausgangsspannung negativ, so gelten entsprechend angepasste Schaltbereiche von -4 bis -1 für Alternative a) und 0 bis 4 für Alternative b) der Schaltregel. - Die entsprechende Schaltregel für den Fall eines Spannungsabfalls ist in der rechten Spalte von Figur 7 dargestellt ist. Auch hier gelten die Alternativen a) und b), jedoch mit angepassten Bereichen wie aus der Figur 7 ersichtlich.

Weiter kann der Modulator 33 ein Hysteresemodul 35 umfassen. Es ist dazu ausgebildet, die Schalthäufigkeit bezüglich der Wechselrichter-Zellen hoher Spannung (HVC) der Gruppe II zu erfassen und bei übermäßiger Schaltaktivität deren Schaltvorgänge zu minimieren. Dazu wirkt das Hysteresemodul 35 bspw. auf die Schaltregeln 34 derart ein, dass die Bereichsgrenzen so verändert werden, dass Alternative b) seltener wird.

Die Schaltregeln 34 sowie deren Schaltbereiche können durch ein Adaptionsmodul 36 insbesondere in Abhängigkeit von Betriebsbedingungen des Multilevel-Inverters 4 mit seinen Wechselrichter-Zellen 5 adaptiert werden. Das Adaptionsmodul 36 umfasst eine Überwachungseinheit mit Ausgleichseinheit 38. Sie erfasst den magnetischen Fluss in den einzelnen Wechselrichter-Zellen jeweils der Gruppe I und II und wirkt so auf die Schaltbereiche der Schaltregeln 34 ein. Dadurch können die Schaltbereiche der Schaltregeln 34 dynamisch verändert werden. Wenn der gesamte magnetische Fluss durch die Wechselrichter-Zellen hoher Spannung (HVC) zu hoch ist, werden die Schaltbereiche so verändert, dass ein Einschalten dieser Zellen erst später erfolgt und dass sie früher wieder abgeschaltet werden. Dazu können Parameter B und D verändert werden, wie sie in den modifizierten Schaltregeln 34 gemäß Figur 8 dargestellt sind.

Ist umgekehrt hingegen der magnetische Fluss zu niedrig, so können mittels der Parameter A und C die Schaltbereiche so verändert werden, dass die Wechselrichter-Zellen hoher Spannung (HVC) früher eingeschaltet und erst später wieder abgeschaltet werden. Auf diese Weise kann der magnetische Fluss ausgeglichen und eine Sättigung vermieden werden.

Ferner umfasst das Adaptionsmodul 36 einen optionalen Einschaltzeitmonitor 39. Dieser erfasst gesondert für die Wechselrichter-Zellen niedriger Spannung (LVC) und denen hoher Spannung (HVC) die Zeitdauer einer positiven oder negativen Spannungsabgabe. Werden bestimmte voreinstellte Grenzwerte überschritten, so können die Schaltbereiche dynamisch angepasst werden in ähnlicher Weise wie vorstehend zu magnetischer Sättigung beschrieben. Es kann aber auch vorgesehen sein, dass die entsprechenden hochbelasteten Wechselrichter-Zellen für eine gewisse Zeit abgeschaltet werden.

Die Wirkung des Adaptionsmoduls 36 mit den dynamisch veränderten Schaltbereichen ist in Figuren 9a und 9b dargestellt. Figur 9a zeigt als Ausgangspunkt das Schaltverhalten gemäß den Schaltregeln 34 mit unveränderten Schaltbereichen, wie sie in Figur 7 dargestellt sind. Wird von der Ausgleichseinheit 38 erkannt, dass der magnetische Fluss durch die Wechselrichter-Zellen hoher Spannung (HVC) gemäß Gruppe II zu gering ist, so verschiebt das Adaptionsmodul 36 den Parameter C, beispielsweise um einen Wert von 3. Es ergeben sich dann entsprechend veränderte Schaltbereiche gemäß den modifizierten Schaltregeln, wie sie in Figur 9b dargestellt sind mit dem Parameter C=3. Das bedeutet, dass die Wechselrichter-Zellen hoher Spannung (HVC), wenn sie einmal eingeschaltet wurden, länger eingeschaltet bleiben, also das Wegschalten verzögert wird, wie die Linie m' zeigt. Eine Auswirkung auf das Einschalten ergibt sich hierbei nicht (dies könnte durch Veränderung des Parameters A erfolgen). Die Schaltaktivität der Wechselrichter-Zellen niedriger Spannung (LVC) verändert sich entsprechend, wie die Linie n' zeigt. Im Ergebnis ist somit das Ein- und Ausschaltverhalten der Wechselrichter-Zellen hoher Spannung (HVC) unsymmetrisch in der Weise, dass sie deutlich später abschalten. Sie sind damit länger eingeschaltet, was deren magnetischen Fluss erhöht. Damit erreicht die dynamische Veränderung des Schaltbereichs der Schaltregeln 34 das gewünschte Ziel einer Erhöhung des magnetischen Flusses in den Wechselrichter-Zellen hoher Spannung (HVC).

Ferner ist ein optionaler Spitzendetektor 37 vorgesehen. An ihm ist das Referenzsignal 43 angelegt. Er ist dazu ausgebildet, das Auftreten von Signalspitzen im Referenzsignal 43 zu erkennen, beispielsweise wenn die Amplitude ihren Maximalwert erreicht. Wird dies erkannt, so kann der Spitzendetektor 37 auf den Modulator 33 in der Weise einwirken, dass ein an sich gemäß den Schaltregeln 34 fälliges Schalten von Wechselrichter-Zellen hoher Spannung (HVC) gesperrt ist, und stattdessen eine überzählige Wechselrichter-Zelle niedriger Spannung (LVC) zum Erreichen der letzten Spannungsstufen geschaltet wird. Eine solche optionale überzählige LVC-Wechselrichter-Zelle in Gruppe I ist in Figur 4 mit gestrichelter Linie dargestellt. Auf diese Weise kann man sich zunutze machen, dass dank des Referenzsignals 43 das Amplitudenmaximum bekannt und leicht zu ist. Der Spitzendetektor 37 erkennt dies und wirkt auf den Modulator 33 so ein, um die Schaltvorgänge von Wechselrichter-Zellen 5 hoher Spannung (HVC) nahe der Amplitudenmaxima zu blockieren oder zumindest zu minimieren.

**Patentansprüche**

1. Elektrochirurgie-Generator, der dazu ausgebildet ist eine hochfrequente Wechselspannung an ein elektrochirurgisches Instrument (16) abzugeben, umfassend eine Gleichspannungsversorgung (2) und einen Inverter für Hochspannung, der von der Gleichspannungsversorgung (2) gespeist ist und eine hochfrequente Wechselspannung mit variabler Spannung und Frequenz erzeugt, die an einem Ausgang (14) zum Anschluss des elektrochirurgischen Instruments (16) angelegt ist, **dadurch gekennzeichnet, dass** der Inverter ausgeführt ist als ein von einem Referenzsignal für die abzugebende Spannung gesteuerter Multilevel-Inverter (4) mit mindestens zwei Gruppen (HVC, LVC) von in Reihe geschalteten Wechselrichter-Zellen (5), wobei jede Gruppe mit einer anderen Gleichspannung versorgt ist und wobei die von den Gruppen abgegebenen Spannungen aufsummiert sind zur Abgabe am Ausgang (14).

2. Elektrochirurgie-Generator nach Anspruch 1, **dadurch gekennzeichnet, dass** die Wechselrichter-Zellen in zwei Gruppen zusammengefasst sind, von denen eine erste Gruppe (LVC) mit niedrigerer Gleichspannung versorgt sind als eine andere, zweite Gruppe (HVC).

3. Elektrochirurgie-Generator nach einem der vorangehenden Ansprüche, **dadurch gekennzeichnet, dass** die Wechselrichter-Zellen (5) bipolar ausgeführt sind und mindestens drei verschiedene Ausgangsspannungen ausgeben, die positiv, negativ oder Null sind.

4. Elektrochirurgie-Generator nach einem der vorangehenden Ansprüche, **dadurch gekennzeichnet, dass** zwischen dem Betrag der Spannung, die von den einzelnen Wechselrichter-Zellen einer zweiten Gruppe (HVC) erzeugt ist, und dem Betrag der Spannung, die von den einzelnen Wechselrichter-Zellen einer ersten Gruppe (LVC) erzeugt ist, ein festes Verhältnis besteht, das vorzugsweise ein ganzzahliges Spannungs-Vielfaches ist, und insbesondere mindestens das Vierfache beträgt.

5. Elektrochirurgie-Generator nach einem der vorangehenden Ansprüche, **dadurch gekennzeichnet, dass** zur Ansteuerung der Wechselrichter-Zellen (5) der Gruppen ein Modulator (33) vorgesehen ist, der dazu ausgebildet ist Schalthäufigkeiten von den Wechselrichter-Zellen hoher Spannung (HVC) zu verringern, indem wahlweise eine Betätigung der Wechselrichter-Zellen hoher Spannung (HVC) ersetzt ist durch eine Betätigung einer Mehrzahl von den Wechselrichter-Zellen niedriger Spannung (LVC), wobei vorzugsweise mit dem Modulator (33) ein Stufensteller (31, 31') zusammenwirkt, an den das Referenzsignal (43) angelegt ist und der dazu ausgebildet ist, das Referenzsignal (43) in ein Spannungsstufensignal umzusetzen, das an den Modulator (33) angelegt ist.

6. Elektrochirurgie-Generator nach Anspruch 5, **dadurch gekennzeichnet, dass** der Modulator (33) über ein Freigabesignal betätigt ist, und ein Veränderungsdetektor (32, 32') vorgesehen ist, der dazu ausgebildet ist eine Veränderung des Referenzsignals (43) und/oder Spannungsstufensignals zu erkennen und das Freigabesignal an den Modulator (33) anzulegen.

7. Elektrochirurgie-Generator nach Anspruch 5 oder 6, **dadurch gekennzeichnet, dass** der Modulator (33) weiter dazu ausgebildet ist, anhand von mindestens einem vorgebbaren Parameter die Anzahl der zu schaltenden Wechselrichter-Zellen der zweiten Gruppe (HVC) zu variieren und eine weitere Anzahl der zu schaltenden Wechselrichter-Zellen der ersten Gruppe (LVC) zu bestimmen sowie diese zur Kompensation gegenläufig zu schalten.

8. Elektrochirurgie-Generator nach dem vorangehenden Anspruch, **dadurch gekennzeichnet, dass** der vorgebbare Parameter eine Schalthäufigkeit der Wechselrichter-Zellen der zweiten Gruppe (HVC) umfasst, und der Modulator (33) ausgebildet ist zur Minimierung dieser Schalthäufigkeit, und/oder der vorgebbare Parameter ein Maß für eine Verlustleistung der Wechselrichter-Zellen umfasst, und der Modulator (33) ausgebildet ist durch Betätigung der Wechselrichter-Zellen der zweiten Gruppe (HVC) verursachte Verlustleistung anzugleichen an die durch Betätigung der Wechselrichter-Zellen der ersten Gruppe (LVC) verursachte Verlustleistung.

9. Elektrochirurgie-Generator nach einem der Ansprüche 5 bis 8, **dadurch gekennzeichnet, dass** in dem Modulator (33) alternative Schaltregeln (34) für Spannungsänderungen implementiert sind, die beide zu derselben Spannungsänderung führen aber eine unterschiedliche Anzahl von Wechselrichter-Zellen der zweiten Gruppe (HVC) schalten.

10. Elektrochirurgie-Generator dem vorangehenden Anspruch, **dadurch gekennzeichnet, dass** bei einer Spannungserhöhung gemäß einer der alternativen Schaltregeln (34) die Anzahl der Wechselrichter-Zellen der zweiten Gruppe (HVC) gleich bleibt und eine der Wechselrichter-Zellen der ersten Gruppe (LVC) zugeschaltet wird, oder gemäß der anderen der alternativen Schaltregeln (34) die Anzahl der geschalteten Wechselrichter-Zellen der zweiten Gruppe (HVC) um Eins erhöht und eine Mehrzahl von Wechselrichter-Zellen der ersten Gruppe (LVC) gegenläufig schaltet, wobei diese Mehrzahl dem um Eins verminderten Spannungs-Vielfachen entspricht.

11. Elektrochirurgie-Generator nach Anspruch 9 oder 10, **dadurch gekennzeichnet, dass** bei einer Spannungsverringerung gemäß einer der alternativen Schaltregeln (34) die Anzahl der Wechselrichter-Zellen der zweiten Gruppe (HVC) gleich bleibt und eine der Wechselrichter-Zellen der ersten Gruppe (LVC) abgeschaltet wird, oder gemäß der anderen der alternativen Schaltregeln (34) die Anzahl der geschalteten Wechselrichter-Zellen der zweiten Gruppe (HVC) um Eins verringert und eine Mehrzahl von Wechselrichter-Zellen der ersten Gruppe (LVC) gegenläufig schaltet, wobei diese Mehrzahl dem um Eins verminderten Spannungs-Vielfachen entspricht.

12. Elektrochirurgie-Generator nach einem der Ansprüche 9 bis 11, **dadurch gekennzeichnet, dass** den Schaltregeln (34) jeweils Schaltbereiche zugewiesen sind, wobei die Schaltbereiche vorzugsweise für positive und negative Ausgangsspannung-Polarität verschieden sind, wobei weiter vorzugsweise Grenzen der Schaltbereiche dynamisch im Betrieb veränderlich sind, vorzugsweise in Abhängigkeit von Zustandsgrößen der Wechselrichter-Zellen der zweiten Gruppe (HVC) und der ersten Gruppe (LVC), insbesondere deren jeweiliger Einschaltzeit, magnetischem Fluss und/oder Temperatur.

**13.** Elektrochirurgie-Generator nach einem der Ansprüche 5 bis 12, **dadurch gekennzeichnet, dass** der Modulator (33) dazu ausgebildet ist, bei mehrfachem Wechsel zwischen Spannungserhöhung und -verringerung ein Schalten der Wechselrichter-Zellen der zweiten Gruppe (HVC) zu blockieren, wobei vorzugsweise zusätzliche Wechselrichter-Zellen der ersten Gruppe (LVC) geschaltet werden, wenn die Spannungserhöhung oder -verringerung den Spannungswert der Wechselrichter-Zellen der zweiten Gruppe (HVC) überschreitet.

**14.** Elektrochirurgie-Generator nach einem der vorangehenden Ansprüche, **dadurch gekennzeichnet, dass** eine Überwachungseinheit vorgesehen ist, die dazu ausgebildet ist magnetischen Fluss in den Wechselrichter-Zellen der zweiten Gruppe (HVC) und/oder Wechselrichter-Zellen der ersten Gruppe (LVC) zu ermitteln und einen so ermittelten Wert für den magnetischen Fluss zu speichern, wobei vorzugsweise eine Ausgleicheinheit (38) vorgesehen ist, die mit der Überwachungseinheit zusammenwirkt und so ausgebildet ist, dass sie bei einer Spannungserhöhung zuerst Wechselrichter-Zellen der zweiten Gruppe (HVC) oder Wechselrichter-Zellen der ersten Gruppe (LVC) mit niedrigem magnetischen Fluss schaltet, und bei einer Spannungsverringerung zuerst solche der Wechselrichter-Zellen mit hohem magnetischen Fluss schaltet.

**15.** Elektrochirurgie-Generator nach einem der vorangehenden Ansprüche, **dadurch gekennzeichnet, dass** ein Steuersignalgenerator (40) für den Multilevel-Inverter (4) vorgesehen ist, der dazu ausgebildet ist ein Referenzsignal (43) für die Ansteuerung des Multilevel-Inverters (4) zu erzeugen, wobei das Referenzsignal (43) ein Muster für von dem Elektrochirurgie-Generator (1) abzugebende Wechselspannung ist, insbesondere hinsichtlich Amplitude, Frequenz, Kurvenform und/oder Tastverhältnis, wobei vorzugsweise die Kurvenform frei wählbar einstellbar ist.

**Claims**

**1.** Electrosurgical generator that is designed to output a high-frequency AC voltage to an electrosurgical instrument (16), comprising a DC voltage supply (2) and a high-voltage inverter that is fed from the DC voltage supply (2) and generates a high-frequency AC voltage having a variable voltage and frequency that is applied to an output (14) for the connection of the electrosurgical instrument (16), **characterized in that** the inverter is designed as a multilevel inverter (4) controlled by a reference signal for the voltage to be output and having at least two groups (HVC, LVC) of series-connected inverter cells (5), wherein each group is supplied with a different DC voltage and wherein the voltages output by the groups are summed to be output at the output (14).

**2.** Electrosurgical generator according to Claim 1, **characterized in that** the inverter cells are combined into two groups, of which a first group (LVC) are supplied with a lower DC voltage than a different, second group (HVC).

**3.** Electrosurgical generator according to either of the preceding claims, **characterized in that** the inverter cells (5) have a bipolar configuration and output at least three different output voltages, which are positive, negative or zero.

**4.** Electrosurgical generator according to one of the preceding claims, **characterized in that** there is a fixed ratio, which is preferably an integer voltage multiple, and in particular at least four times, between the absolute value of the voltage that is generated by the individual inverter cells of a second group (HVC) and the absolute value of the voltage that is generated by the individual inverter cells of a first group (LVC).

**5.** Electrosurgical generator according to one of the preceding claims, **characterized in that**, in order to drive the inverter cells (5) of the groups, provision is made for a modulator (33) that is designed to reduce switching frequencies of the high-voltage inverter cells (HVC) by selectively replacing actuation of the high-voltage inverter cells (HVC) with actuation of a plurality of the low-voltage inverter cells (LVC), wherein a tap changer (31, 31') preferably interacts with the modulator (33), to which tap changer the reference signal (43) is applied and which tap changer is designed to convert the reference signal (43) into a voltage level signal that is applied to the modulator (33).

**6.** Electrosurgical generator according to Claim 5, **characterized in that** the modulator (33) is actuated via an enable signal, and provision is made for a change detector (32, 32') that is designed to identify a change in the reference signal (43) and/or voltage level signal and to apply the enable signal to the modulator (33).

**7.** Electrosurgical generator according to Claim 5 or 6, **characterized in that** the modulator (33) is furthermore designed to vary the number of inverter cells of the second group (HVC) to be switched based on at least one predefinable parameter and to determine a further number of the inverter cells of the first group (LVC) to be switched and to switch these in an opposing manner for compensation purposes.

**8.** Electrosurgical generator according to the preceding claim, **characterized in that** the predefinable parameter comprises a switching frequency of the inverter cells of the second group (HVC), and the modulator (33) is designed to minimize this switching frequency, and/or the predefinable parameter comprises a metric for power loss of the inverter cells, and the modulator (33) is designed to adapt power loss caused by actuating the inverter cells of the second group (HVC) to the power loss caused by actuating the inverter cells of the first group (LVC).

**9.** Electrosurgical generator according to one of Claims 5 to 8, **characterized in that** alternative switching rules (34) for voltage changes are implemented in the modulator (33), these both leading to the same voltage change but switching a different number of inverter cells of the second group (HVC).

**10.** Electrosurgical generator according to the preceding claim, **characterized in that**, in the event of a voltage increase, according to one of the alternative switching rules (34), the number of inverter cells of the second group (HVC) remains the same and one of the inverter cells of the first group (LVC) is activated, or according to the other of the alternative switching rules (34), the number of switched inverter cells of the second group (HVC) is increased by one and a plurality of inverter cells of the first group (LVC) are switched in an opposing manner, wherein this plurality corresponds to the voltage multiple minus one.

**11.** Electrosurgical generator according to Claim 9 or 10, **characterized in that**, in the event of a voltage decrease, according to one of the alternative switching rules (34), the number of inverter cells of the second group (HVC) remains the same and one of the inverter cells of the first group (LVC) is deactivated, or according to the other of the alternative switching rules (34), the number of switched inverter cells of the second group (HVC) is reduced by one and a plurality of inverter cells of the first group (LVC) are switched in an opposing manner, wherein this plurality corresponds to the voltage multiple minus one.

**12.** Electrosurgical generator according to one of Claims 9 to 11, **characterized in that** respective switching ranges are assigned to the switching rules (34), wherein the switching ranges are preferably different for positive and negative output voltage polarity, wherein furthermore preferably limits of the switching ranges are dynamically changeable during operation, preferably on the basis of state variables of the inverter cells of the second group (HVC) and the inverter cells of the first group (LVC), in particular their respective switch-on time, magnetic flux and/or temperature.

**13.** Electrosurgical generator according to one of Claims 5 to 12, **characterized in that** the modulator (33) is designed, in the case of multiple changing between voltage increase and decrease, to block switching of the inverter cells of the second group (HVC), with preferably additional inverter cells of the first group (LVC) being switched when the voltage increase or decrease exceeds the voltage value of the inverter cells of the second group (HVC).

**14.** Electrosurgical generator according to one of the preceding claims, **characterized in that** provision is made for a monitoring unit that is designed to ascertain magnetic flux in the inverter cells of the second group (HVC) and/or the inverter cells of the first group (LVC) and to store a value thus ascertained for the magnetic flux, wherein provision is preferably made for a compensation unit (38) that interacts with the monitoring unit and is designed such that, in the event of a voltage increase, it first switches inverter cells of the second group (HVC) or inverter cells of the first group (LVC) with a low magnetic flux and, in the event of a voltage decrease, first switches those of the inverter cells with a high magnetic flux.

**15.** Electrosurgical generator according to one of the preceding claims, **characterized in that** provision is made for a control signal generator (40) for the multilevel inverter (4), which is designed to generate a reference signal (43) for driving the multilevel inverter (4), wherein the reference signal (43) is a pattern for AC voltage to be output by the electrosurgical generator (1), in particular with regard to amplitude, frequency, curve form and/or duty cycle, wherein the curve form is preferably able to be set freely as desired.

**Revendications**

**1.** Générateur électrochirurgical, conçu pour délivrer une tension alternative à haute fréquence à un instrument électrochirurgical (16), ledit générateur comprenant une alimentation en tension continue (2) et un inverseur à haute tension qui est alimenté par l'alimentation en tension continue (2) et qui génère une tension alternative à haute fréquence, de tension et fréquence variables, qui est appliquée à une sortie (14) destinée au raccordement de l'instrument électrochirurgical (16), **caractérisé en ce que** l'inverseur est conçu comme un inverseur à niveaux multiples (4) commandé par un signal de référence pour la tension à délivrer et comprenant au moins deux groupes (HVC, LVC) de cellules d'onduleur (5) montées en série, chaque groupe étant alimenté par une tension continue différente et les tensions délivrées par les groupes étant additionnées pour être délivrées à la sortie (14).

**2.** Générateur électrochirurgical selon la revendication 1, **caractérisé en ce que** les cellules d'onduleur sont regroupées en deux groupes dont un premier groupe (LVC) est alimenté avec une tension continue inférieure à celle d'un deuxième groupe différent (HVC).

**3.** Générateur électrochirurgical selon l'une des revendications précédentes, **caractérisé en ce que** les cellules d'onduleur (5) sont conçues pour être bipolaires et délivrent au moins trois tensions de sortie différentes qui sont positives, négatives ou nulles.

**4.** Générateur électrochirurgical selon l'une des revendications précédentes, **caractérisé en ce que**, entre la valeur de la tension qui est générée par les cellules d'onduleur individuelles d'un deuxième groupe (HVC) et la valeur de la tension qui est générée par les cellules d'onduleur individuelles d'un premier groupe (LVC), il existe un rapport fixe qui est de préférence un multiple de tension entier, et en particulier au moins le quadruple.

**5.** Générateur électrochirurgical selon l'une des revendications précédentes, **caractérisé en ce que**, pour commander les cellules d'onduleur (5) des groupes, un modulateur (33) est prévu qui est conçu pour réduire les fréquences de commutation de cellules d'onduleur à haute tension (HVC) en remplaçant sélectivement l'actionnement des cellules d'onduleur à haute tension (HVC) par l'actionnement d'une pluralité de cellules d'onduleur à basse tension (LVC), le modulateur (33) coopérant de préférence avec un actionneur à échelons (31, 31') auquel le signal de référence (43) est appliqué et qui est conçu pour convertir le signal de référence (43) en un signal d'échelon de tension qui est appliqué au modulateur (33).

**6.** Générateur électrochirurgical selon la revendication 5, **caractérisé en ce que** le modulateur (33) est actionné par le biais d'un signal de libération, et un détecteur de variation (32, 32'), qui est conçu pour détecter une variation du signal de référence (43) et/ou du signal de niveau de tension et appliquer le signal de libération au modulateur (33).

**7.** Générateur électrochirurgical selon la revendication 5 ou 6, **caractérisé en ce que** le modulateur (33) est en outre conçu pour faire varier le nombre de cellules d'onduleur du deuxième groupe (HVC) à commuter en fonction d'au moins un paramètre spécifiable et pour déterminer un autre nombre de cellules d'onduleur du premier groupe (LVC) à commuter et à les commuter dans des sens opposés pour effectuer la compensation.

**8.** Générateur électrochirurgical selon la revendication précédente, **caractérisé en ce que** le paramètre spécifiable comprend une fréquence de commutation des cellules d'onduleur du deuxième groupe (HVC), et le modulateur (33) est conçu pour minimiser cette fréquence de commutation, et/ou le paramètre spécifiable comprend une mesure d'une perte de puissance des cellules d'onduleur, et le modulateur (33) est conçu pour ajuster la perte de puissance provoquée par l'actionnement des cellules d'onduleur du deuxième groupe (HVC) à la perte de puissance provoquée par l'actionnement des cellules d'onduleur du premier groupe (LVC).

**9.** Générateur électrochirurgical selon l'une des revendications 5 à 8, **caractérisé en ce que** des règles de commutation alternatives (34) pour les variations de tension sont mises en œuvre dans le modulateur (33), lesquelles règles conduisent toutes deux à la même variation de tension mais à un nombre différent de cellules d'onduleur du deuxième groupe (HVC).

**10.** Générateur électrochirurgical selon la revendication précédente, **caractérisé en ce que**, lorsque la tension augmente selon l'une des règles de commutation alternatives (34), le nombre de cellules d'onduleur du deuxième groupe (HVC) reste le même et l'une des cellules d'onduleur du premier groupe (LVC) est activé, ou, selon l'autre des règles de commutation alternatives (34), le nombre de cellules d'onduleur commutées du deuxième groupe (HVC) est augmenté d'une et une pluralité de cellules d'onduleur du premier groupe (LVC) commute dans des sens opposées, cette pluralité correspondant au multiple de tension réduit de un.

**11.** Générateur électrochirurgical selon la revendication 9 ou 10, **caractérisé en ce que**, lorsque la tension est réduite selon l'une des règles de commutation alternatives (34), le nombre de cellules d'onduleur du deuxième groupe (HVC) reste le même et l'une des cellules d'onduleur du premier groupe (LVC) est désactivée ou, selon l'autre des règles de commutation alternatives (34), le nombre de cellules d'onduleur commutées du deuxième groupe (HVC) est réduit d'une et une pluralité de cellules d'onduleur du premier groupe (LVC) commutent dans des sens opposés, cette pluralité correspondant au multiple de tension réduit de un.

**12.** Générateur électrochirurgical selon l'une des revendications 9 à 11, **caractérisé en ce que** les règles de commutation (34) sont chacune affectées à des zones de commutation, les zones de commutation étant de préférence différentes pour la polarité de tension de sortie positive et négative, de préférence des limites des zones de commutation étant en outre modifiables dynamiquement pendant le fonctionnement, de préférence en fonction de grandeurs d'état des cellules d'onduleur du deuxième groupe (HVC) et du premier groupe (LVC), en particulier de leur temps d'activation respectif, de leur flux magnétique et/ ou leur température.

13. Générateur électrochirurgical selon l'une des revendications 5 à 12, **caractérisé en ce que** le modulateur (33) est conçu pour bloquer la commutation des cellules d'onduleur du deuxième groupe (HVC) en cas de passages multiples entre une augmentation de tension et une diminution, de préférence des cellules d'onduleur supplémentaires du premier groupe (LVC) étant commutées lorsque l'augmentation ou la diminution de tension devient supérieure à la valeur de tension des cellules d'onduleur du deuxième groupe (HVC).

14. Générateur électrochirurgical selon l'une des revendications précédentes, **caractérisé en ce qu'**une unité de surveillance est prévue qui est conçue pour déterminer le flux magnétique dans les cellules d'onduleur du deuxième groupe (HVC) et/ou les cellules d'onduleur du premier groupe (LVC) et mémoriser une valeur ainsi déterminée pour le flux magnétique, de préférence une unité de compensation (38) étant prévue qui coopère avec l'unité de surveillance et qui est conçue pour, lorsque la tension augmente, commuter d'abord les cellules d'onduleur du deuxième groupe (HVC) ou les cellules d'onduleur du premier groupe (LVC) qui ont un flux magnétique faible, et, lorsque la tension est réduite, commuter d'abord les cellules de d'onduleur qui ont un flux magnétique élevé.

15. Générateur électrochirurgical selon l'une des revendications précédentes, **caractérisé en ce qu'**un générateur de signal de commande (40) est prévu pour l'inverseur à niveaux multiples (4), lequel générateur est conçu pour générer un signal de référence (43) destiné à commander l'inverseur à niveaux multiples (4), le signal de référence (43) étant un modèle pour la tension alternative à délivrer par le générateur électrochirurgical (1), notamment en ce qui concerne l'amplitude, la fréquence, la forme d'onde et/ou le rapport cyclique, de préférence la forme d'onde pouvant être librement ajustée.

**Fig. 1**

a)

b)

**Fig. 2**

Fig. 3

Fig. 4a)

Fig. 4b)

Fig. 5

a)

b)

Fig. 6

| $V_{out}$ | a) LVC only | b) HVC + LVC | a) LVC only | b) HVC + LVC |
|---|---|---|---|---|
| ≥0 | [-4; 3] | [4; 4] | [1; 4] | [-4; 0] |
| <0 | [-4; -1] | [0; 4] | [-3; 4] | [-4; -4] |

**Fig. 7**

| $V_{out}$ | a) LVC only | b) HVC + LVC | a) LVC only | b) HVC + LVC |
|---|---|---|---|---|
| ≥0 | [-4; 3-A] | [4-A; 4] | [1-C; 4] | [-4; 0-C] |
| <0 | [-4; -1+B] | [0+B; 4] | [-3+D; 4] | [-4; -4+D] |

**Fig. 8**

a)

b)

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2514380 B1
- EP 3216409 A1